# EUROPÄISCHE PATENTSCHRIFT

(11) **EP 1 281 405 B1**
(45) Veröffentlichungstag und Bekanntmachung des Hinweises auf die Patenterteilung: **21.01.2009**
(21) Anmeldenummer: 02090268.0
(22) Anmeldetag: 28.03.2000
(51) Int. Cl.: A61K 49/00, C09B 23/02, C09B 23/00, C09B 23/08

(54) **Kurzkettige Peptid-Farbstoffkonjugate als Kontrastmittel für die optische Diagnostik**
Short-chained peptide-dye conjugates as contrast agents for optical diagnosis
Conjugués de peptides à courte chaîne avec des colorants comme agent de contraste pour le diagnostique optique

(30) Priorität: 09.04.1999 DE 19917713
(43) Veröffentlichungstag der Anmeldung: 05.02.2003
(62) Teilanmeldung aus: 00922560.8
(73) Patentinhaber: Institut für Diagnostikforschung GmbH an der Freien Universität Berlin, 13353 Berlin (DE)
(72) Erfinder: Licha, Kai, 14612 Falkensee (DE); Becker, Andreas, 85570 Markt Schwaben (DE); Semmler, Wolfhard, c/o Deutsches Krebsforschungsz., 69120 Heidelberg (DE); Wiedemann, Bertram, 14195 Berlin (DE); Hessenius, Carsten, 12049 Berlin (DE); Volkmer-Engert, Rudolf, 13349 Berlin (DE); Schneider-Mergener, Jens, 10717 Berlin (DE); Bhargava, Sarah, 10711 Berlin (DE)
(74) Vertreter: Seuss, Thomas

(56) Entgegenhaltungen:
- WO-A-00/21576
- WO-A-90/06949
- WO-A-97/40104
- WO-A-98/47538
- DE-A- 19 649 971
- US-A- 5 382 654
- US-A- 5 753 205
- US-A- 5 824 772
- REUBI J -C: "THE ROLE OF PEPTIDES AND THEIR RECEPTORS AS TUMOR MARKERS" ENDOCRINOLOGY AND METABOLISM CLINICS OF NORTH AMERICA,US,W.B. SAUNDERS COMPANY, PHILADELPHIA, Bd. 22, Nr. 4, 1. Dezember 1993 (1993-12-01), Seiten 917-939, XP000578548 ISSN: 0889-8529
- LICHA K ET AL: "Highly parallel nano-synthesis of cleavable peptide-dye conjugates on cellulose membranes" TETRAHEDRON LETTERS,ELSEVIER SCIENCE PUBLISHERS, AMSTERDAM,NL, Bd. 41, Nr. 11, März 2000 (2000-03), Seiten 1711-1715, XP004202255 ISSN: 0040-4039
- JAEHDE ULRICH ET AL: "Quantification and visualization of the transport of octreotide, a somatostatin analogue, across monolayers of cerebrovascular endothelial cells." PHARMACEUTICAL RESEARCH (NEW YORK), Bd. 11, Nr. 3, 1994, Seiten 442-448, XP000992868 ISSN: 0724-8741
- BECKER, ANDREAS ET AL: "Novel receptor-targeted contrast agents for optical imaging of tumors" PROC. SPIE-INT. SOC. OPT. ENG., VOL. 3924, NO. MOLECULAR IMAGING: REPORTERS, DYES, MARKERS, AND INSTRUMENTATION, PAGE(S) 41-47, 23. - 24. Januar 2000, XP000992960
- BECKER, ANDREAS ET AL: "Cyanine dye labeled vasoactive intestinal peptide and somatostatin analog for optical detection of gastroenteropancreatic tumors" ANN. N. Y. ACAD. SCI., 2000, VOL. 921, NO. VIP, PACAP, GLUCAGON, AND RELATED PEPTIDES, PAGE(S) 275-278, XP000992937

## Beschreibung

Die Erfindung betrifft Verbindungen zur Tumordiagnostik, bestehend aus Konjugaten von Farbstoffen mit kurzkettigen Peptiden, die vom vasoaktiven intestinalen Peptid, vom Somatostatin oder vom Neurotensin abgeleitet sind, die Verwendung dieser Verbindungen als optische Diagnostika, und diese Verbindungen enthaltende diagnostische Mittel.

Krankheitsbedingte Veränderungen spiegeln sich auf zellulärer Ebene oft in einer gegenüber dem Normalzustand veränderten Rezeptorverteilung oder -expression wieder. Diese Unterschiede können sowohl quantitativer Art (z. B. Menge der Transferrin-Rezeptoren auf proliferierenden Zellen) oder aber auch qualitativer Art sein (z. B. Expression von Vascular endothelial growth factors, VEGF). Bisher werden Ansätze zur Abbildung einer pathologischen Rezeptorexpression oder -verteilung wegen der notwendigen Sensitivität des Detektionsverfahrens hauptsächlich in der Radiodiagnostik verfolgt.

Heptahelikale Rezeptoren sind Zielmoleküle von vielen pharmakologischen Wirkstoffen (z. B. β-Blocker, H2-Säure-Blocker, Antihistaminika). Neben therapeutischen Ansätzen werden hauptsächlich radioaktiv markierte agonistische Liganden dieser Rezeptoren diagnostisch für die sog. Rezeptorszintigraphie zur In-vivo-Detektion und Lokalisation von Tumoren eingesetzt. Hierbei wird der Mechanismus der rezeptorvermittelten Endozytose, z. B. durch den Somatostatinrezeptor, der auf neuroendokrinen Tumoren vermehrt exprimiert ist, ausgenutzt. Klinisch zugelassen für die szintigraphische Routinediagnostik ist das Somatostatinanalogon ¹¹¹In-DTPA-pentetreotid (Octreoscan^{®}); Literatur: J. Steroid Biochem. Mol. Biol. 37, 1079-82, 1990, J. Nucl. Med. 32, 1184-9, 1991,; J. Nucl. Med. 33, 652-8, 1992; Digestion 3, 54-9, 1994, J. Clin. Invest. 93, 1321-5, 1994, Metabolism 45, 21-3, 1996.

Ein anderer Ansatz besteht in der Nutzung von radioaktiv markiertem VIP und VIP-Analoga, welche an den VIP-Rezeptor binden. Der VIP-Rezeptor wird von einem breiten Spektrum von Tumoren vermehrt exprimiert (u. a. Adenokarzinome).
WO 96/30055 beschreibt radiodiagnostische und radiotherapeutische Reagenzien, speziell VIP-rezeptorbindende Peptide, die radioaktiv markiert sind und für die Radiodiagnostik und -therapie eingesetzt werden können. Mit besonderem Vorteil beschrieben sind VIP-rezeptorbindende, mit Tc-99m markierbare Peptide für die Szintigraphie. Weitere Literatur: Cancer Research 54, 690-700, 1994; Endocrinology 136, 2662-80, 1994, J. Nucl. Med. 40, 353-361, 1999.

Alle beschriebenen diagnostischen Ansätze, die auf dem Somatostatinrezeptor und VIP-Rezeptor basieren, sind radiodiagnostische Ansätze (Szintigraphie mit ¹²³I, ¹²⁵I, ¹¹¹In oder ^{99m}Tc-markierten Peptiden).
Literatur: EP 588754, US 5650134; US 5620675; US 5225180; WO 96/23527; J. Steroid Biochem. Mol. Biol. 37, 1083-87, 1990; Lancet 242-4, 1989, J. Nucl. Med. 39, 1913-17, 1998.

Bisher sind jedoch keine fluoreszenzmarkierten Peptide bekannt, die mit Farbstoffen konjugiert sind, die eine In-vivo-Fluoreszenzdetektion von Tumoren ermöglichen (Photochem. Photobiol. 68, 603-632, 1998).

Der Erfindung liegt die Aufgabe zu Grunde, neue Verbindungen zur Verfügung zu stellen, die eine sensitive Diagnose von Tumoren durch Detektion von Fluoreszenzstrahlung unter Ausnutzung einer rezeptorspezifischen Bindung der Verbindungen an das Zielgewebe ermöglichen. Hierbei sollen spezielle Farbstoffmoleküle, die an Biomoleküle gekoppelt sind, ein hochsensitiv detektierbares Fluoreszenzsignal liefern.

Die Aufgabe wird durch die Bereitstellung von Verbindungen, die Fluoreszenzfarbstoffe enthalten, welche kovalent an kurzkettige Peptide gekoppelt sind, gelöst. Diese Konjugate besitzen eine hohe Bindungsaffinität zu heptahelikalen Rezeptoren, speziell dem Somatostatinrezeptor, dem VIP-Rezeptor (Vasoaktives intestinales Peptid) und dem Neurotensinrezeptor, und werden ggf. durch rezeptorvermittelte Endozytose intrazellulär aufgenommen. Die erfindungsgemäßen Verbindungen sind daher geeignet für die technisch einfache, unschädliche optische Diagnostik von Tumorzellen und Tumorgeweben, die Somatostatinrezeptoren VIP-Rezeptoren oder Neurotensinrezeptoren im Vergleich zu gesunden Zellen erhöht exprimieren. Insbesondere geeignet sind die Verbindungen für die Fluoreszenzdiagnostik und mit besonderem Vorteil für die fluoreszenzendoskopische Diagnostik in Hohlorganen, wie dem Ösophagus, der Zervix, des Kolons und der Bronchien von verschiedenen Tumortypen, wie z. B. Adenokarzinomen, neuroendokrinen Tumoren oder duktalen pankreatischen Tumoren.

Besonders bevorzugte Farbstoffe zeichnen sich dadurch aus, daß sie bestimmte photophysikalische und chemische Anforderungen erfüllen. Aus photophysikalischer Sicht müssen die Farbstoffe hohe Absorptionskoeffizienten und hohe Fluoreszenzquantenausbeuten besitzen, um ein effektives Signal auch bei geringsten Gewebekonzentrationen zu erzeugen. Die Absorptionsmaxima müssen frei wählbar einem weiten spektralen Bereich überdecken. So ist für die Detektion in tieferen Gewebeschichten (mehrere Zentimeter unter der Oberfläche) der Spektralbereich zwischen 600 und 900 nm essentiell, während für oberflächliche Detektion Absorptionswellenlängen von 400 bis 600 nm ausreichen. Aus chemischer Sicht müssen die Farbstoffe eine hohe Photostabilität besitzen und keine Zersetzungserscheinungen (Photobleaching) während der Anregung aufweisen. Die Farbstoffe müssen als Synthesebaustein in der festphasensynthetischen Herstellung der Peptide einsetzbar sein, und somit unter den gängigen Synthesebedingungen stabil sein, damit eine einfache, günstige Produktion strukturell definierter Farbstoff-Peptid-Konjugate mit festem stöchiometrischen Verhältnis zwischen Farbstoff und Peptid gewährleistet ist. Die Anforderungen werden am besten von Polymethinfarbstoffen, insbesondere Cyanin-, Merocyanin-, Oxonol- und Squariliumfarbstoffen erfüllt.

### Gegenstand der Erfindung sind daher

Peptid-Polymethinfarbstoff-Konjugate der allgemeinen Formel (I)

**A¹ - (X)ₘ - A²** **I**

worin
- X: für eine α, β oder γ-Aminosäure mit D oder L-Konfiguration und
- m: für eine Zahl von 5 bis 30 steht,
wobei die resultierende Aminosäuresequenz (X)ₘ geradkettiger Natur oder über eine Disulfidbrücke zwischen zwei Cysteinen oder Homocysteinen oder amidisch zwischen N- und C-Terminus cyclisiert sein kann und für die Aminosäuresequenz des Somatostatins oder für Fragmente, Teilsequenzen, Derivate oder A-naloga des Somatostatins steht,
- A¹: für ein Wasserstoffatom, einen Acetylrest oder einen Alkylrest mit bis zu 10 C-Atomen, der gegebenenfalls mit 1 bis 3 Carboxygruppen und/oder 1 bis 6 Hydroxygruppen substituiert sein kann, oder einen Poly(oxyethylen)rest mit 2 bis 30 -CH₂CH₂O-Einheiten oder ein Farbstoffmolekül aus der Klasse der Polymethinfarbstoffe, welches mindestens ein Absorptionsmaximum im Bereich von 380 bis 1200 nm aufweist, steht,
- A²: für eine Hydroxygruppe, eine Aminogruppe oder ein Farbstoffmolekül aus der Klasse der Polymethin-farbstoffe, welches mindestens ein Absorptionsmaximum im Bereich von 380 bis 1200 nm aufweist, steht
unter der Bedingung, daß mindestens einer der Reste A¹ oder A² ein Farbstoffmolekül aus der Klasse der Polymethinfarbstoffe, welches mindestens ein Absorptionsmaximum im Bereich von 380 bis 1200 nm aufweist, darstellt,
wobei für den Fall, daß A¹ und/oder A² ein Farbstoffmolekül aus der Klasse der Polymethinfarbstoffe, welches mindestens ein Absorptionsmaximum im Bereich von 380 bis 1200 nm aufweist, darstellen, A¹ an die N-terminale Aminogruppe und A² an eine Aminogruppe der Aminosäure Lysin oder an eine Hydroxygruppe der Aminosäure Serin in beliebiger Position innerhalb der Aminosäuresequenz (X)ₘ geknüpft ist,
Wobei Farbstoffmolekül aus der Klasse der Polymethinfarbstoffe für einen Cyaninfarbstoff der allgemeinen Formel II 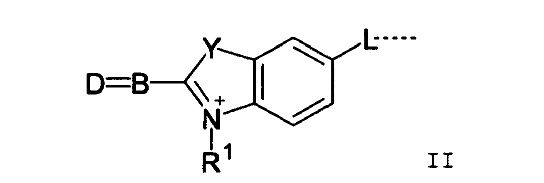 worin
D für ein Fragment entsprechend den allgemeinen Formeln III bis VI, wobei die mit einem Stern gekennzeichnete Position die Verknüpfung mit B bedeutet, steht, 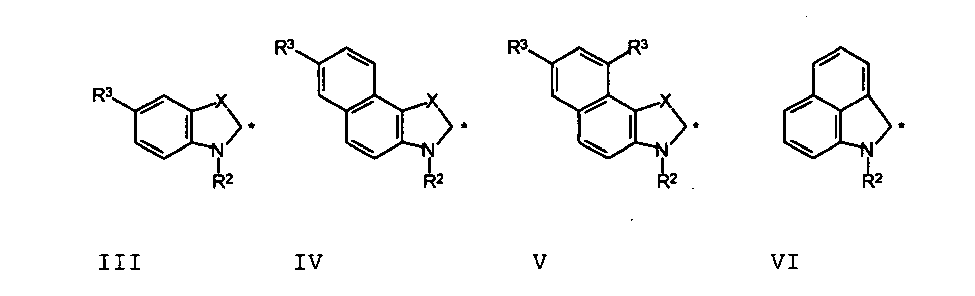 B für ein Fragment entsprechend den allgemeinen Formeln VII bis XII 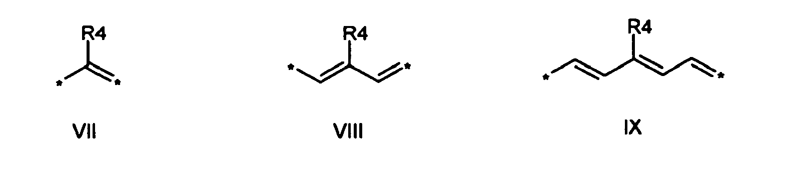 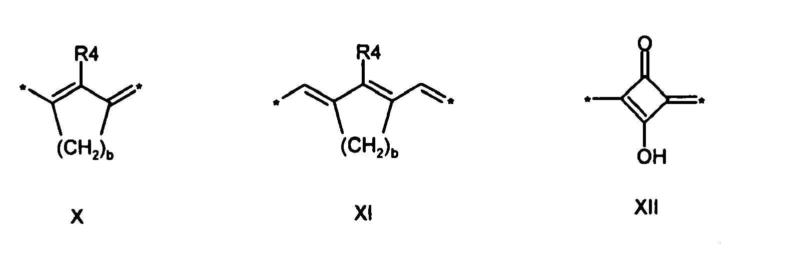 R¹ und R² für E¹, R³ für ein Fluor-, Chlor-, Brom-, Iodatom oder eine Nitrogruppe oder für einen Rest - COOE¹, -CONE¹E², -NHCOE¹, -NHCONHE¹, -NE¹E², -OE¹, - OSO₃E¹, -SO₃E¹, -SO₂NHE¹, -E¹,
wobei E¹ und E² unabhängig voneinander für ein Wasserstoffatom, eine C₁-C₄-Sulfoalkylkette, eine gesättigte oder ungesättigte, verzweigte oder geradkettige C₁-C₅₀-Alkylkette, wobei die Kette oder Teile dieser Kette gegebenenfalls eine oder mehrere aromatische oder gesättigte zyklische C₅-C₆- oder bizyklische C₁₀-Einheiten formen können, steht, und wobei die C₁-C₅₀-Alkylkette von 0 bis 15 Sauerstoffatomen und/oder von 0 bis 3 Carbonylgruppen unterbrochen ist und/oder mit 0 bis 5 Hydroxygruppen substituiert ist, stehen,
R⁴ für ein Wasserstoffatom, für ein Fluor-, Chlor-, Brom-, Iodatom oder eine verzweigte oder geradkettige C₁-C₁₀-Alkylkette, steht,
b eine Zahl 2 oder 3 bedeutet,
X und Y unabhängig voneinander O, S, Se, -CH=CH- oder C(CH₃)₂ bedeuten,
L für eine Gruppe entsprechend nachstehender Formeln 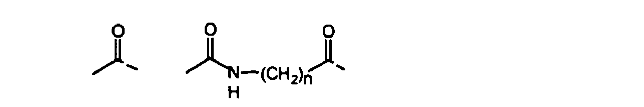 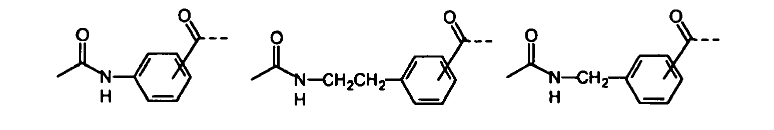
worin n eine Zahl von 1 bis 10 bedeutet,
steht,
und deren physiologisch verträgliche Salze.

Fragmente, Teilsequenzen, Derivate oder Analoga der genannten Peptide stehen u. a. für verkürzte Aminosäuresequenzen, Austausche einzelner oder sämtlicher Aminosäuren gegen die entsprechenden D-Aminosäuren, Austausche einzelner Aminosäuren gegen andere Aminosäuren, invertierte Sequenzen und Kombinationen der genannten Merkmale.

Die Fragmente, Teilsequenzen, Derivate oder Analoga der genannten Peptide können auch nicht natürliche Aminosäuren, wie z.B. Naphthalanin, Cyclohexylalanin, Norleucin, Norvalin, α-Aminoadipinsäure, α-Aminobuttersäure, β-Alanin, β-Cyclohexlyalanin, Ornithin, Sarcosin oder δ-Hydroxylysin, enthalten.

(Bei den obengenannten Formeln stellt die im Molekülteil links gezeichnete durchgezogene Linie die Verknüpfung zum Farbstoffgrundgerüst und die unterbrochene Linie im Molekülteil rechts die Verknüpfung zum Peptid dar.)

Aus der Klasse der Polymethinfarbstoffe sind die Cyaninfarbstoffe, z. B. die auf der Indolstruktur basierenden Indocarbo-, Indodicarbo- und Indotricarbcyanine besonders vorteilhaft. Diese Strukturen zeichnen sich durch eine hohe chemische und photochemische Stabilität aus. Durch günstige Synthese sind Derivate erhältlich, die beliebig zwischen 400 und 1000 nm absorbieren und fluoreszieren, durch Substitution mit geeigneten Linkern und funktionellen Gruppen, vorzugsweise Carboxylgruppen, an Peptide gekoppelt werden können und eine hohe Wasserlöslichkeit, vorzugsweise durch Sulfonatgruppen, besitzen. Im Gegensatz zu literaturbekannten Cyaninfarbstoffen besitzen die erfindungsgemäß verwendeten Verbindungen nur eine reaktive Gruppe, die eine stöchiometrisch definierte Kopplung an das Peptid im Zuge der Harzsynthese des Konjugates ermöglicht.

Besonders bevorzugte Ausführungsformen der erfindungsgemäßen Verbindungen der allgemeinen Formel I zeichnen sich daher dadurch aus,
- daß das Farbstoffmolekül A¹ und/oder A² für einen Indocarbocyanin-, einen Indodicarbocyanin- oder einen Indotricarbocyaninfarbstoff steht,
- daß das Farbstoffmolekül A¹ und/oder A² für einen Indocarbocyanin-, einen Indodicarbocyanin- oder einen Indotricarbocyaninfarbstoff der allgemeinen Formel XIII oder XIV 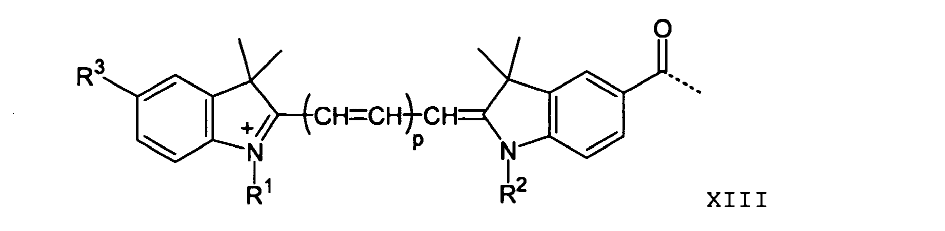 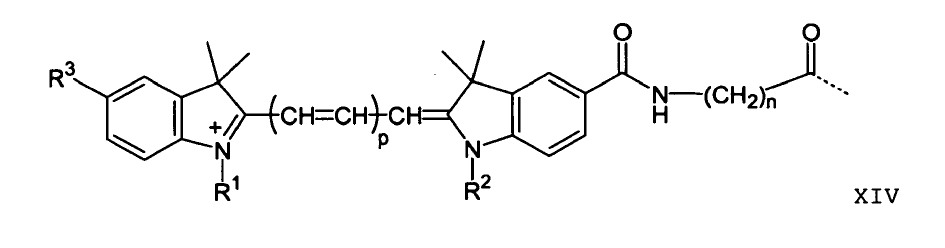 worin
   p für 1, 2 oder 3,
   n für eine Zahl von 1, 2, 3, 4 oder 10 steht,
   R¹ und R² unabhängig voneinander für einen 4-Sulfobutyl-, 3-Sulfopropyl-, 2-Sulfoethyl-, 3-Methyl-3-sulfopropyl-, Methyl-, Ethyl- oder Propylrest stehen, und
   R³ für Wasserstoff, ein Chlor-, Brom-, Iodatom oder eine Nitrogruppe oder für einen Rest -COOE¹, -CONE¹E², -NHCOE¹, -NHCONHE¹, -NE¹E², -OE¹, -OSO₃E¹, -SO₃E¹, -SO₂NHE¹,
   wobei E¹ und E² unabhängig voneinander für ein Wasserstoffatom oder für einen Methyl-, Ethyl- oder einen C₃-C₆-Alkylrest, der von 0 bis 2 Sauerstoffatomen und/oder von 0 bis 1 Carbonylgruppen unterbrochen ist und/oder mit 0 bis 5 Hydroxygruppen substituiert ist, oder für einen Poly(oxyethylen)glykolrest mit 2 bis 30 -CH₂CH₂O-Einheiten stehen, steht,
- daß das Farbstoffmolekül A¹ und/oder A² für einen Indocarbocyanin-, einen Indodicarbocyanin- oder einen Indotricarbocyaninfarbstoff der allgemeinen Formel XIII oder XIV 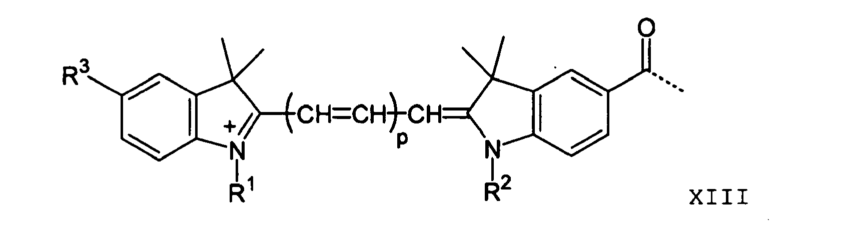 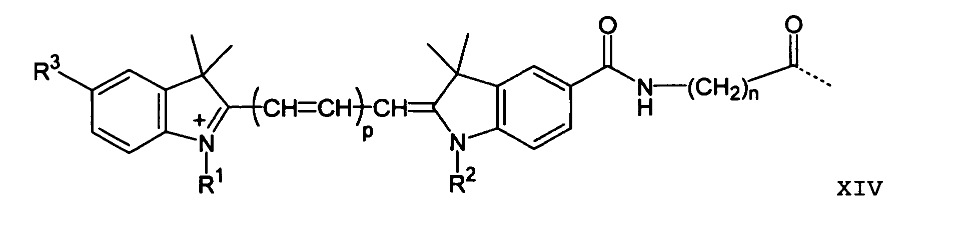 worin
   p für 1, 2 oder 3,
   n für 1, 2 oder 4,
   R¹ und R² unabhängig voneinander für einen 4-Sulfobutyl- oder 3-Sulfopropylrest,
   R³ für Wasserstoff oder für einen Rest -COOE¹ oder -CONHE¹,
   wobei E¹ ein Wasserstoffatom oder ein Methyl-, Ethyloder ein C₃-C₆-Alkylrest, der von 0 bis 2 Sauerstoffatomen und/oder von 0 bis 1 Carbonylgruppen unterbrochen ist und/oder mit 0 bis 5 Hydroxygruppen substituiert ist, bedeutet,
   steht,
- daß das Farbstoffmolekül A¹ und/oder A² für einen Indotricarbocyaninfarbstoff der allgemeinen Formel XV oder XVI steht: 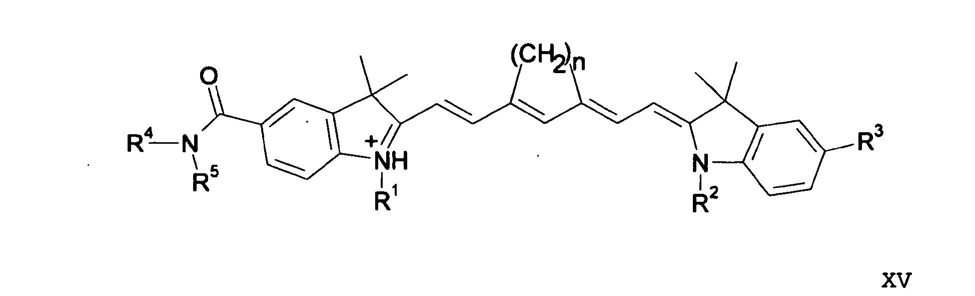 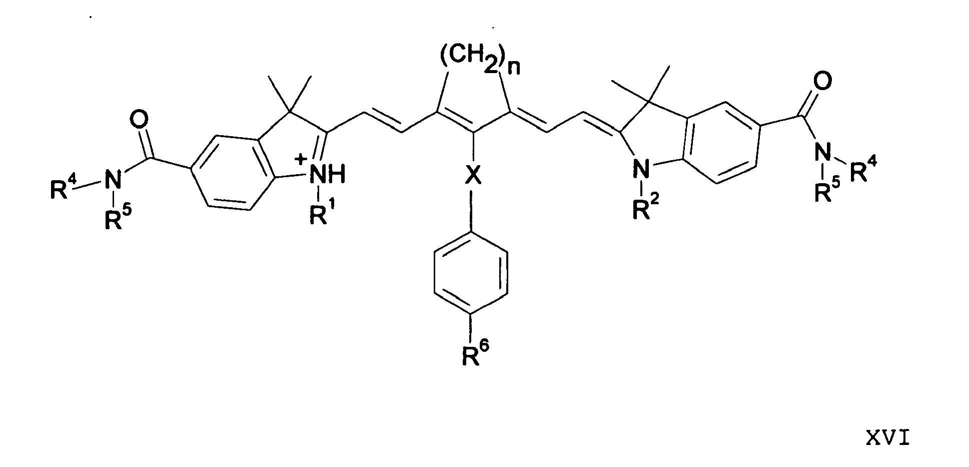 worin
   n für 2 oder 3 steht,
   R¹ und R² unabhängig voneinander einen 4-Sulfobutyl-, 3-Sulfopropyl- oder 2-Sulfoethylrest darstellen,
   R³ für einen Rest -CONH-Peptid, -CONH-(CH₂)ₘ-CONH-Peptid, -CONH-(CH₂)ₙ-NH-CS-NH-Peptid oder -CONH-(CH₂)ₙ-NHCO-CH₂-Peptid mit m = 1 bis 10 und n = 2 oder 3 steht,
   oder eine folgende Gruppe darstellt: 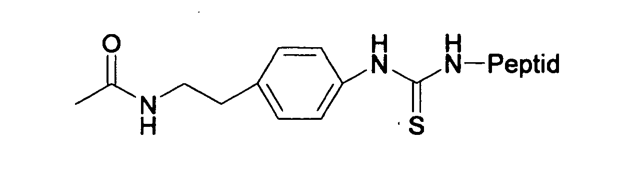 R⁴ und R⁵ unabhängig voneinander für ein Wasserstoffatom, einen Methylrest oder einen hydroxylierten Alkylrest wie z.B. 2-Hydroxyethyl, 3-Hydroxypropyl, 2,3-Dihydroxypropyl, 1,3-Dihydroxy-2-propyl, 2,3,4-Trihydroxybutyl, 1,3,4-Trihydroxy-2-butyl, 2,3,4,5,6-Pentahydroxyhexyl stehen,
   R⁶ für eine der folgenden Gruppen steht:
   -(CH₂)ₘ-CONH-Peptid mit m = 0 bis 2,
   -(CH₂)ₘ-NH-CS-NH-Peptid mit m = 0 bis 2,
   und X für ein Sauerstoffatom oder ein Schwefelatom steht;
- daß das Farbstoffmolekül A¹ und/oder A² für einen Indotricarbocyaninfarbstoff der allgemeinen Formel XVII steht: 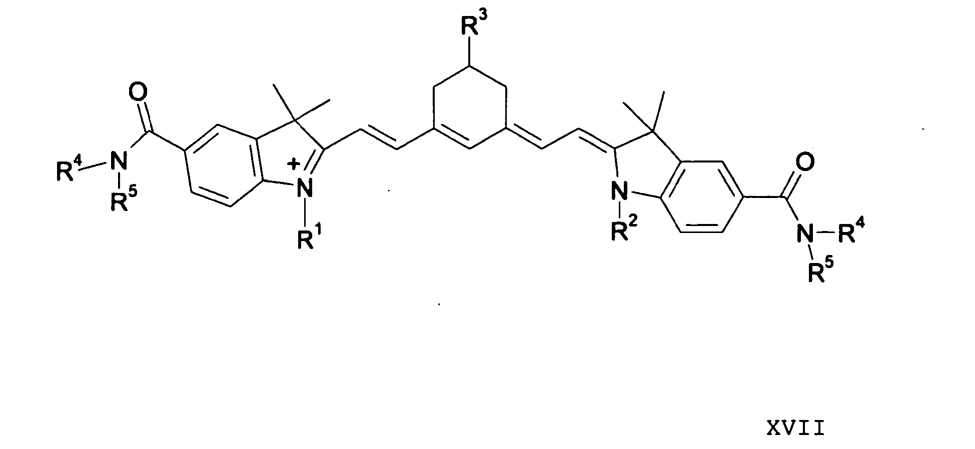
worin
R¹ und R² unabhängig voneinander für einen 4-Sulfobutyloder 3-Sulfopropylrest darstellen,
R³ für einen Rest -CONH-Peptid, -CONH-(CH₂)ₘ-CONH-Peptid, -CONH-(CH₂)ₙ-NH-CS-NH-Peptid oder -CONH-(CH₂)ₙ-NHCO-CH₂-Peptid mit m = 1 bis 10 und n = 2 oder 3 steht, oder eine folgende Gruppe darstellt: 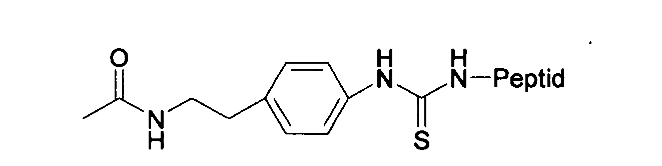 und R⁴ und R⁵ unabhängig voneinander für ein Wasserstoffatom, einen Methylrest oder einen hydroxylierten Alkylrest wie z.B. 2-Hydroxyethyl, 3-Hydroxypropyl, 2,3-Dihydroxypropyl, 1,3-Dihydroxy-2-propyl, 2,3,4-Trihydroxybutyl, 1,3,4-Trihydroxy-2-butyl, 2,3,4,5,6-Pentahydroxyhexyl stehen.

Die Verwendung von farbstoffmarkierten Antikörpern zur Tumordetektion ist literaturbekannt (J. Cell. Pharmacol. 3, 141-145, 1992; Cancer Immunol. Immunother. 41, 257-63, 1995; Cancer Research 54, 2643-9, 1994, Biotechnol. Prog. 13, 649-658, 1997).
Im Gegensatz dazu enthalten die erfindungsgemäßen Verbindungen als Biomoleküle niedermolekulare Peptide und Peptidderivate, die die Vorteile von Antikörpern, wie eine hohe Bindung an Zielstrukturen, aufweisen, ohne daß das diagnostische Potential durch eine ungünstige Pharmakokinetik (lange Bluthalbwertszeiten, allergene Nebeneffekte (Immunogenität)) eingeschränkt ist.

Biologische und pharmakologische Anforderungen an Peptidsequenzen sind dementsprechend eine ausreichende Plasmastabilität bei schneller Anreicherung im Zielgewebe und gleichzeitiger schneller Eliminierung aus dem Restkörper, vorzugsweise über den renalen Ausscheidungsweg.

Weitere besonders bevorzugte erfindungsgemäße Verbindungen der allgemeinen Formel I zeichnen sich dadurch aus,
- daß (X)ₘ für die Aminosäuresequenz des Somatostatins entsprechend 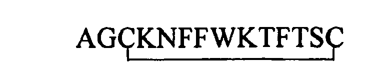 oder für Fragmente, Teilsequenzen, Derivate oder Analoga des Somatostatins, bestehend aus 5 bis 20 Aminosäuren,
   steht,
- daß 2 bis m Aminosäuren unabhängig voneinander gegen ihre jeweilige D-Aminosäure oder gegen andere L- oder D-Aminosäuren ausgetauscht sein können, wobei m die oben angegebene Bedeutung hat,
- daß mindestens eine der Aminosäuren (X)ₘ unabhängig voneinander gegen andere, nicht natürliche Aminosäuren oder Aminosäurederivate ausgetauscht sein kann,
- daß mindestens eine der Aminosäuren (X)ₘ unabhängig voneinander gegen andere, nicht natürliche Aminosäuren oder Aminosäurederivate, wie z. B. Naphthalanin, Cyclohexylalanin, Norleucin, Norvalin, α-Aminoadipinsäure, α-Aminobuttersäure, β-Alanin, β-Cyclohexlyalanin, Ornithin, Sarcosin oder δ-Hydroxylysin, ausgetauscht sein kann,
- daß sämtliche Aminosäuren (X)ₘ gegen ihre jeweilige D-Aminosäure ausgetauscht sind,
- daß als Fragmente, Teilsequenzen, Derivate oder Analoga des Somatostatins folgende Aminosäuresequenzen ausgewählt sind:

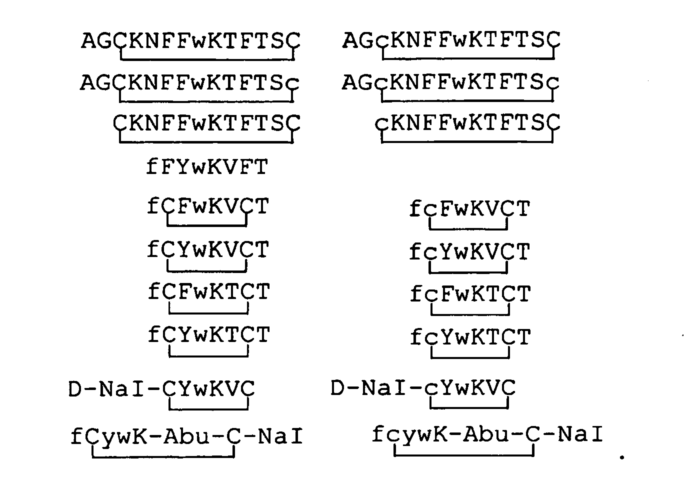

Die Terminologie der allg. Formel I beinhaltet die übliche Schreibweise von Aminosäuresequenzen. Der N-Terminus ist stets links und der C-Terminus rechts (unsubstituiert entsprechend H-(X)ₘ-OH oder, im Fall eines Amids, H-(X)ₘ-NH₂). Die verwendeten Einbuchstaben-Abkürzungen der Aminosäuren können nachgelesen werden in M. Bodanszky, Peptide chemistry - A practical textbook, 2nd edition, Springer-Verlag Heidelberg 1993, S. 3. Großbuchstaben bedeuten Aminosäuren mit L-Konfiguration (natürliche Aminosäuren), Kleinbuchstaben bedeuten D-Aminosäuren, Disulfidbrücken (cyclische Peptide) sind durch Verbindungstriche zwischen den entsprechenden Buchstaben (C = Cystein oder Homocystein) gekennzeichnet. Mit retrosynthetisch bezeichnet man Sequenzen, bei denen in der Synthese die Reihenfolge der Aminosäuren im Vergleich zu einer gegebenen nativen Sequenz invertiert ist, d. h. man beginnt die Synthese mit der ursprünglich N-terminalen Aminosäure und erzeugt die Sequenz bis zur ursprünglich C-terminalen Aminosäure.

Die erfindungsgemäßen Substanzen haben verschiedene Vorteile gegenüber radioaktiv markierten Substanzen. Fluoreszenzfarbstoffe können beliebig oft zur Fluoreszenzemission angeregt werden. Es liegt kein kontinuierliches Signal vor, das einem Zerfall mit entsprechender Halbwertszeit zu Grunde liegt. Dementsprechend ist der Zeitpunkt der Diagnose beliebig wählbar und beliebig oft wiederholbar und nicht durch die Halbwertszeit eines Isotops limitiert. Der Patient ist keiner ionisierenden Strahlung ausgesetzt, die verwendete Lichtstrahlung ist in den verwendeten Dosen unschädlich. Die optische Detektionstechnik erlaubt die hochsensitive Detektion weniger Photonen und ist daher bezüglich der Sensitivität mit der Radiodiagnostik vergleichbar.

Ein Verfahren zur Diagnostik mittels Nahinfrarotstrahlung (NIR-Strahlung) unter Verwendung von Farbstoffbiomolekülkonjugaten wurde beschrieben (WO 96/17628). Als besonders vorteilhaft erwies sich im Falle der vorliegenden Erfindung, daß die Peptidkonjugate in günstiger Ausbeute und hoher Reinheit durch das Verfahren der automatisierten Festphasensynthese darstellbar sind. Überraschenderweise wurde gefunden, daß verschiedene Carboxylgruppentragende Indocyaninverbindungen als Aminosäureanaloga eingesetzt und sowohl N-terminal als auch an eine Aminogruppe des Lysins an der Festphase (Harz) gekoppelt werden können. Nach Abspaltung vom Harz und chromatographischer Reinigung wurden die Farbstoff-Peptid-Konjugate in Reinheiten > 95% erhalten.

Das wesentliche Problem bei Nutzung von Licht zur Fluoreszenzanregung ist die begrenzte Eindringtiefe des Lichtes, die im VIS im Submillimeterbereich liegt, im NIR jedoch Zentimeter betragen kann. Hinsichtlich der Eindringtiefe unproblematisch sind Detektionsverfahren in oberflächlichen Gewebeerkrankungen, sowie weichen Geweben.

Erfindungsgegenstand sind daher mammographische Verfahren, endoskopische Verfahren und intraoperative Verfahren, bei denen unter Verwendung der erfindungsgemäßen Verbindungen erkrankte Gewebebereiche durch Detektion der Fluoreszenz oder der nicht absorbierten Strahlung diagnostiziert werden. Besonderer Erfindungsgegenstand ist die Verwendung der erfindungsgemäßen Verbindungen in endoskopischen Verfahren, z. B. die Koloskopie, Bronchoskopie, Oesophagialendoskopie, bei denen oberflächennahe Gewebeveränderungen diagnostiziert werden. Die Verwendung von Weißlicht mit direkter visueller Beurteilung ist in der endoskopischen Diagnostik weit verbreitet. Die erfindungsgemäßen Verbindungen tragen durch die Erzeugung eines gewebespezifischen Signals zu einer entscheidenden Verbesserung des Verfahrens bei, insbesondere bei der Diagnostik frühzeitiger, visuell nicht erfaßbarer Gewebeveränderungen (z. B. Dysplasien des Kolons).

Es wurde gefunden, daß sich nach Versprühen der erfindungsgemäßen farbstoffgekoppelten Verbindungen im Darm von Ratten mit chemisch induzierten Dysplasien und Kolonkarzinomen, anschließendem Spülen und der Durchführung einer endoskopischen Fluoreszenzdiagnostik (Anregung 740 nm, Detektion oberhalb 760 nm) Gewebebereiche erhöhter Fluoresenz im Kolon nachweisen ließen.

Gegenstand der Erfindung ist daher auch ein Verfahren zur endoskopischen Fluoreszenzdiagnostik, insbesondere des Gastrointestinaltraktes, unter Verwendung der erfindungsgemäßen Verbindungen. Dabei wird dem Gewebe eine oder mehrere der Substanzen, vorzugsweise intravenös, oder topisch durch Versprühen zugeführt und Licht aus dem entsprechenden Spektralbereich zur elektronischen Anregung des verwendeten Farbstoffes eingestrahlt. Die reflektierte oder die vom Farbstoff emittierte Fluoreszenzstrahlung wird registriert. Bevorzugt sind die Methoden, bei denen das Gewebe großflächig bestrahlt und die Fluoreszenzstrahlung örtlich aufgelöst durch Aufnahme mit einer CCD-Kamera dargestellt wird oder die abzubildenden Gewebeareale mit einem Lichtleiter abgerastert und die erhaltenen Signale rechnerisch in ein synthetisches Bild umgesetzt werden. Dabei kann die Fluoreszenz spektral und/oder phasenselektiv sowie stationär und/oder zeitaufgelöst detektiert und ausgewertet werden. Die erhaltenen Fluoreszenzbilder können simultan mit Weißlichtbildern erzeugt werden und zur Datenauswertung in einer Abbildung übereinander dargestellt werden.

Die Synthese der erfindungsgemäßen Verbindungen erfolgt in Anlehnung an literaturbekannte Methoden. Die Peptide werden festphasensynthetisch an Polymerharzen hergestellt. Einzelheiten sind dem Fachmann bekannt.
Literatur: Peptide chemistry - A practical textbook (M. Bodanszky), 2nd edition, Springer-Verlag Heidelberg 1993; Anti-Cancer Drug Design 12, 145-167, 1997; J. Am. Chem. Soc. 117, 11821-2, 1995.

Die Farbstoffe werden separat hergestellt und dann im Zuge der festphasensynthetischen Darstellung der Peptide an diese gekoppelt und die erfindungsgemäßen Verbindungen nach Abspaltung vom Harz und Reinigung als hochreine Verbindungen erhalten. Bevorzugt sind solche Farbstoffe, die Carboxylgruppen enthalten, welche nach Aktivierung mittels gängiger Reagenzien mit Aminogruppen des Peptides, insbesondere der ε-Aminogruppe des Lysins oder des N-terminalen Peptidaminogruppe, gekoppelt werden. Weiterhin bevorzugt sind Farbstoffe mit Halogenalkyl- oder Halogenacetylresten, die an Thiolgruppen des Peptides, insbesondere der Aminosäure Cystein oder Homocystein, gekoppelt werden.
Literatur zur Synthese von Polymethinfarbstoffen: Bioconjugate Chem. 4, 105-111, 1993; Bioconjugate Chem. 7, 356-62, 1996; Bioconjugate Chem. 8, 751-56, 1997; Cytometry 10, 11-19, 1989 und 11, 418-30, 1990; J. Heterocycl. Chem. 33, 1871-6, 1996; J. Org. Chem. 60, 2391-5, 1995; Dyes and Pigments 17, 19-27, 1991, Dyes and Pigments 21, 227-34, 1993; J. Fluoresc. 3, 153-155, 1993; Anal. Biochem. 217, 197-204, 1994; US 4981977; US 5688966; US 5808044; WO 97/42976; WO 97/42978; WO 98/22146; WO 98/26077; EP 0800831.

Der Vorteil nur einer aktivierbaren Gruppe, wie z.B. einer Carboxylgruppe, oder einer bereits aktivierten Gruppe, wie z.B. einem Isothiocyanat, einer Halogenalkylgruppe oder einer Halogenacetylgruppe, besteht darin, daß eine chemisch einheitliche Kopplung erfolgen kann. Die Halogenacetylgruppe hat den besonderen Vorteil, daß eine chemisch einheitliche Kopplung an die Mercaptogruppe des Cysteins oder Homocysteins erfolgt. Diese Kopplung kann in Lösung an das ungebundene und von Schutzgruppen befreite Peptid erfolgen. Durch die aktivierten Gruppen ist eine Kopplung an Peptide möglich, ohne daß Nebenreaktionen auftreten. Zur Erhöhung der Wasserlöslichkeit weisen die Peptid-Farbstoffkonjugate am Farbstoff eine erhöhte Anzahl von Hydroxygruppen auf. Durch die Position des Linkers am Indolsystem des Farbstoffes kann zusätzlich eine ausreichende Hydrophilie durch Sulfonatgruppen enthaltende Reste an den Stickstoffatomen des Indolsystems erzeugt werden. Dadurch kann eine strukturell einheitliche Kopplungsreaktion mit den Peptiden (siehe Beispiele 4 bis 12, 28 bis 32 und 44 bis 49) durchgeführt werden.

Ein weiterer Gegenstand der Erfindung ist ein optisches Diagnostikum zur In-vivo-Diagnostik erkrankter Gewebebereiche, welches sich dadurch auszeichnet, daß es mindestens eine Verbindung der allgemeinen Formel I zusammen mit den üblichen Hilfs- und/oder Trägerstoffen sowie Verdünnungsmitteln enthält.

Folgende Beispiele erläutern die Erfindung:

### Beispiele 1 bis 3: Synthese der Indocyaninfarbstoffe 1-3 zur festphasensynthetischen Kopplung an Aminogruppen (N-terminal oder ε-Lysin) der Peptide

Die Synthese erfolgt generell ausgehend von 1-(4-Sulfobutyl)-2,3,3-trimethyl-*3H*-indolenin und 1-(4-Sulfobutyl)-2,3,3-trimethyl-5-carboxy-*3H*-indolenin (Cytometry 10, 11-19, 1989, Talanta 39, 505-510, 1992).

### Beispiel 1: Synthese von 1,1'-Bis(4-sulfobutyl)indocarbocyanin-5-carbonsäure, Natriumsalz (1)

0,8 g ( 4,0 mmol) N,N-Diphenylformamidin werden in 15 ml Essigsäureanhydrid vorgelegt und bei Raumtemp. portionsweise mit 1,4 g (4,2 mmol) 1-(4-Sulfobutyl)-2,3,3-trimethyl-5-carboxy-*3H*-indolenin versetzt, 30 min bei 120 °C gerührt und dann mit einem Wasserbad auf Raumtemp. abgekühlt. Anschließend werden 1,2 g (4,1 mmol) 1-(4-Sulfobutyl)-2,3,3-trimethyl-*3H*-indolenin, 1,2 g (14,6 mmol) wasserfreies Natriumacetat, 15 ml Essigsäureanhydrid und 6 ml Essigsäure zugegeben. Das Reaktionsgemisch wird 1 h auf 120 °C erhitzt, die tiefrote Lösung abgekühlt und mit 100 ml Ether versetzt. Der ausgefallene Feststoff wird abfiltriert. Es erfolgt eine chromatographische Reinigung an RP-Kieselgel EUROPREP 60-30 C18, 60A, 20-45 m (Eluens: Wasser/MeOH, Stufengradient von 0 % auf 70 % MeOH). Die produktenthaltenden Fraktionen werden am Rotationsverdampfer vom Methanol befreit und anschließend lyophilisiert, Ausbeute: 1,5 g (58 %), rotes Lyophilisat.

### Beispiel 2:

### Synthese von 1,1'-Bis(4-sulfobutyl)indodicarbocyanin-5-carbonsäure, Natriumsalz (2)

1,2 g (4,1 mmol) 1-(4-Sulfobutyl)-2,3,3-trimethyl-3*H-*indolenin und 1,0 g ( 3,9 mmol) Malonaldehyd-bis-phenylimin-hydrochlorid werden in 15 ml Essigsäureanhydrid 30 min bei 120 °C gerührt und dann mit einem Wasserbad auf Raumtemp. abgekühlt. Anschließend werden nacheinander 1,4 g (4,2 mmol) 1-(4-Sulfobutyl)-2,3,3-trimethyl-5-carboxy-*3H*-indolenin, 1,2 g (14,6 mmol) wasserfreies Natriumacetat, 15 ml Essigsäureanhydrid und 6 ml Essigsäure zugegeben. Das Reaktionsgemisch wird 1 h auf 120 °C erhitzt, die nun blaue Lösung abgekühlt und mit 100 ml Ether versetzt. Die Aufarbeitung und Reinigung erfolgt wie in Beispiel 1 beschrieben, Ausbeute: 1,8 g (66 %), blaues Lyophilisat.

### Beispiel 3:

### Synthese von 1,1'-Bis(4-sulfobutyl)indotricarbocyanin-5-carbonsäure, Natriumsalz (3)

1,2 g (4,1 mmol) 1-(4-Sulfobutyl)-2,3,3-trimethyl-*3H-*indolenin und 1,1 g ( 3,9 mmol) Glutaconaldehyddianilhydrochlorid werden in 15 ml Essigsäureanhydrid 30 min bei 120 °C gerührt und dann mit einem Wasserbad auf Raumtemp. abgekühlt. Anschließend werden 1,4 g (4,2 mmol) 1-(4-Sulfobutyl)-2,3,3-trimethyl-5-carboxy-3*H-*indolenin, 1,2 g (14,6 mmol) wasserfreies Natriumacetat, 15 ml Essigsäureanhydrid und 6 ml Essigsäure zugegeben. Das Reaktionsgemisch wird 1 h auf 120 °C erhitzt, die nun blaue Lösung abgekühlt und mit 100 ml Ether versetzt. Die Aufarbeitung und Reinigung erfolgt wie in Beispiel 1 beschrieben, Ausbeute: 1,8 g (60 %), blaues Lyophilisat.

### Beispiele 4 bis 6: Synthese der Indocyaninfarbstoffe 4-6 aus 1-3 zur festphasensynthetischen Kopplung an Aminogruppen (N-terminal oder ε-Lysin) der Peptide

Die Synthese erfolgt durch Amidierung der Farbstoffe 1-3 mit β-Alanin-t-butylester und saurer Spaltung der t-Butylestergruppe.

Eine Lösung von 0,5 mmol des Farbstoffes **1-3** und 0,1 g (1,0 mmol) Triethylamin in 20 ml Dimethylformamid wird bei 0 °C mit 0,5 mmol TBTU in 10 ml Dimethylformamid versetzt und 15 min bei 0 °C gerührt. Anschließend wird eine Lösung von 0,11 g (0,6 mmol) β-Alanin-t-butylesterhydrochlorid und 0,6 mmol Triethylamin in 5 ml Dimethylformamid zugetropft und das Reaktionsgemisch 2 h bei Raumtemp. gerührt. Nach Zugabe von 100 ml Diethylether wird der ausgefallene Feststoff abfiltriert, in 20 ml Dichlormethan gelöst, mit 10 ml Trifluoressigsäure versetzt und 24 h bei Raumtemp. gerührt. Das Gemisch wird im Vakuum eingeengt und der Rückstand wie in Beispiel 1 beschrieben chromatographisch gereinigt und lyophilisiert; Ausbeuten: 0,21 g (58%) **4,** 0,29 g (76%) **5,** 0,28 g (72%) **6.**

### Beispiele 7 bis 9: Synthese der Indocyaninfarbstoffe 7-9 aus 1-3 zur festphasensynthetischen Kopplung an Aminogruppen (N-terminal oder ε-Lysin) der Peptide

Die Darstellung erfolgt analog der Beispiele 4-6 unter Verwendung von 0,1 g (0,6 mmol) Glycin-t-butylesterhydrochlorid, Ausbeuten: 0,25 g (68%) **4,** 0,30 g (80%) **5,** 0,32 g (83%) **6**.

### Beispiele 10 bis 12: Synthese der Indocyaninfarbstoffe 10-12 aus 1-3 zur festphasensynthetischen Kopplung an Peptide durch Kopplung an Thiolgruppen von Cystein.

Die Synthese erfolgt durch Amidierung der Farbstoffe 1-3 mit 3-Aminopropanol und anschließender Überführung der Alkoholgruppe in ein Bromid.

Eine Lösung von 0,5 mmol des Farbstoffes **1-3** und 0,1 g (1,0 mmol) Triethylamin in 20 ml Dimethylformamid wird bei 0 °C mit 0,5 mmol TBTU in 10 ml Dimethylformamid versetzt und 15 min bei 0 °C gerührt. Anschließend wird eine Lösung von 850 mg (1,2 mmol) 3-Aminopropanol und 1,2 mmol Triethylamin in 5 ml Dimethylformamid zugetropft und das Reaktionsgemisch 6 h bei Raumtemp. gerührt. Nach Zugabe von 100 ml Diethylether wird der ausgefallene Feststoff abfiltriert und wie in Beispiel 1 beschrieben chromatographisch gereinigt und lyophilisiert.
Die Umsetzung zu den Bromiden **10-12** erfolgt durch Rühren von 0,3 mmol der Zwischenprodukte mit 55 mg (0,5 mmol) N-Bromsuccinimid und 130 mg (0,5 mmol) Triphenylphosphin in einem Gemisch von 4 ml Dichlormethan und 4 ml Dimethylformamid für 48 h bei 4 °C. Durch Zugabe von 3 ml Ether werden die Produkte ausgefällt, abfiltriert und als Rohprodukte in der Peptidkopplung eingesetzt.

### Beipiele 28 bis 32: Harzsynthese von Peptidkonjugaten bestehend aus somatostatinrezeptorbindenden Peptiden und den Farbstoffen 1-9 und 13.

Allgemeine Vorschrift:
Die Synthesen wurden an 500 mg TCP-Thr(But)-fmoc-Harz (Fa. Pepchem Tübingen) mit einer Beladung von 0,49 mmol/g durchgeführt. Das Peptid wird "step by step" unter Verwendung folgender temporärer Schutzgruppen synthetisiert: tert.-butyl für Thr und Ser, Trityl für Cys und Asp, Boc für Trp und Lys. Als Kondensationsreagenz wird HBTU verwendet.

Nach Abspaltung der N-terminalen Fmoc-Schutzgruppe wird der Farbstoff **1-9, 13** in einem gesonderten Syntheseschritt kondensiert. Dazu wird 255 mg Harz in ca. 2 ml DMF suspendiert und mit 0,5 mmol Farbstoff, 0,5 mmol HBTU und 0,17 ml DIEA versetzt. Es wird 18 h bei Raumtemp. gerührt, das Harz abgesaugt, mit Dichlormethan gewaschen und getrocknet. Die Abspaltung des Farbstoff-Peptidkonjugates vom Harz erfolgte mit 95% Trifluoressigsäure unter Zusatz von Triisopropylsilan mit anschließender Lyophilisierung aus 10% Essigsäure. Das Rohprodukt wird mittels Aktivkohle cyclisiert und chromatographisch gereinigt (50x300mm VYDAC RP-18, Gradient: Wasser/Acetonitril).

Die Strukturen der synthetisierten Farbstoff-Peptid-Konjugate sind in der folgenden Übersicht zusammengefaßt:

### Farbstoffkonjugate mit Somatostatinrezeptorbindenden Peptiden

### Beispiel 28 bis 30: Konjugat von Farbstoff 1-3 mit Pentetreotid

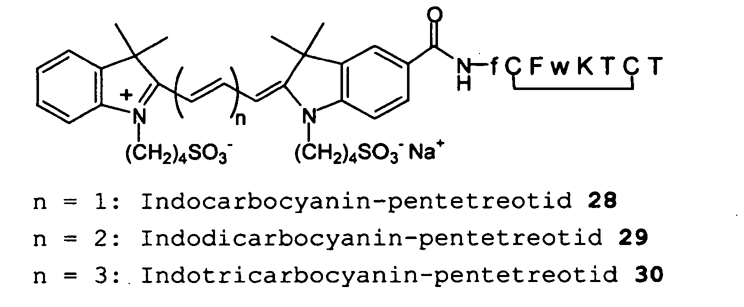

### Beispiel 31: Konjugat von Farbstoff 3 mit Somatostatin-14

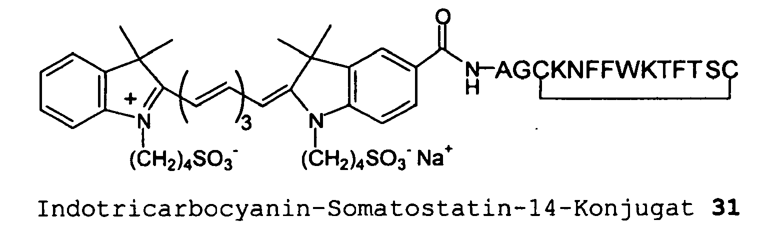

### Beispiel 32: Konjugat von Farbstoff 13 mit Somatostatin-14

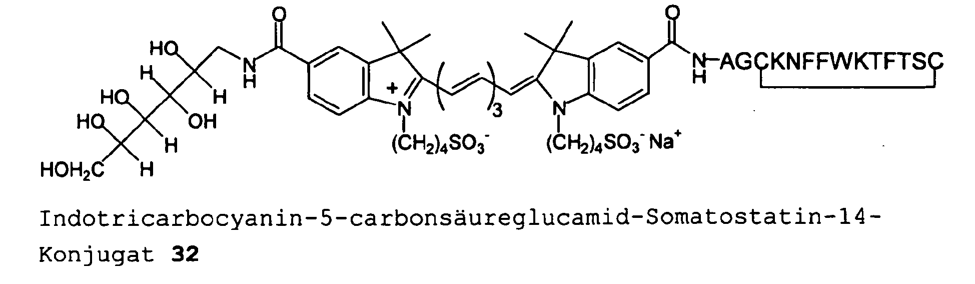

### Beispiel 39: Absorptions- und Fluoreszenzeigenschaften der synthetisierten Farbstoff-Peptidkonjugate

Absorptionsmaxima und Extinktionskoeffizienten wurden in PBS und in Rinderplasma bestimmt (Perkin Elmer Lambda 2). Fluoreszenzemissionsspektren wurden in PBS durch Anregung auf der kurzwelligen Seite (ca. 40 nm vom Absorptionsmaximum) erhalten (SPEX Fluorolog, R928 PMT).

### Beispiel 40: Bestimmung der Zellaufnahme mittels Fluoreszenzmikroskopie

Die Bindung und Aufnahme der erfindungsgemäßen Verbindungen wurden in vitro an humanen Tumorzellen untersucht, die Rezeptoren für vasoaktives intestinales Peptid und/oder Somatostatin und/oder Neurotensin exprimieren. Dazu wurden 5 x 10⁵ Tumorzellen in 1,5 ml Medium inkubiert, welches die Testsubstanz enthielt. Es wurden unterschiedliche Konzentrationen der Substanzen (10 nM-10 µM) eingesetzt und die Inkubationsdauer variiert (1 min-24 h). Nach der Inkubation wurden die Zellen fixiert und mikroskopische Präparate hergestellt. Die Auswertung erfolgte an einem Zeiss Axiovert 135-Fluoreszenzmikroskop, das mit einem Cy 7- (Exciter HQ 710/70nm, Emitter 810/90nm, Beamsplitter 750nm LP), Cy5- (Exciter 575-625 nm, Emitter 660-710nm BP, Beamsplitter 645nm) und Cy3-Filtersatz (Exciter 546/12nm, Emitter 590nm LP, Beamsplitter 580nm) ausgestattet war. Von allen Präparaten wurden Weißlicht- und Fluoreszenzbilder mit einer CCD-Kamera (Visitron RTE/CCD-576) aufgenommen und digital gespeichert.

### Beispiel 42: Untersuchung der Stabilität von Farbstoff-Peptidkonjugaten in Rinderplasma

Die chemische Stabilität der erfindungsgemäßen Verbindungen in Plasma wurde in vitro in Abhängikeit von der Zeit mittels HPLC untersucht. Dazu wurden 1 mM Lösungen der Peptide in PBS in Rinderblutplasma (Fa. Graeber, gefroren, zur Heparinanalyse) unter Erhalt einer Konzentration von 30 µM pipettiert und die Lösungen bei 37 °C inkubiert.
Zu verschiedenen Zeitpunkten (0,5; 1; 2; 4; 6; 24 h) erfolgte die Aufarbeitung der Proben, indem 1 ml der Plasmalösung mit 1 ml MeOH versetzt wird und die ausgefallenen Proteine abzentrifugiert werden.
Die Analyse des Überstandes erfolgte mittels HPLC durch Bestimmung des Gehaltes bei 750 nm bezogen auf den Gehalt nach 1 min Inkubation bei 0 °C (Kontrolle).
HPLC: Beckmann, Diodenarraydetektor TIDAS (Fa. J&M) 350-1000 nm;
Säule: Chromasil 5µ, 250mm x 4,5mm
Laufmittel: A: 90% H₂0(+0.5%TFA)/10% MeOH
B: 10% H₂0(+0.5%TFA)/90% MeOH
Gradient: 10% B auf 100% B innerhalb von 20 min

### Literatur zur Spotsynthese:

1. Frank, R. (1992) Spot synthesis: an easy technique for the positionally addressable, parallel chemical synthesis on a membrane support. Tetrahedron 48, 9217-9232
2. Kramer, A., Schneider-Mergener, J. (1998) Synthesis and screening of peptide libraries on continuos cellulose membrane supports. Methods in Molecular Biology 87, 25-39
3. Volkmer-Engert, R., Hoffmann, B., Schneider-Mergener, J. (1997) Tetrahedron Lett. 38, 1029-1032
4. Licha, K., Bhargava, S., Rheinländer, C., Becker, A., Schneider-Mergener, J., Volkmer-Engert, R. (in press) Highly paralles Nano-synthesis of cleavable peptidedye conjugates on cellulose membranes. Tetrahedron Lett.

### Beispiel 44

### a) 5-N-(2,3-Dihydroxypropyl)aminocarbonyl-1-(4-sulfobutyl)-2,3,3-trimethyl(3H)indolenin

0,9 g (2,6 mmol) 5-Carboxy-1-(4-sulfobutyl)-2,3,3-Trimethyl(3H)indolenin (Anal. Biochem. 217,197, 1994) werden in 30 ml absolutem N,N-Dimethylformamid und 3 ml Pyridin vorgelegt und mit 1,35 g (5,3 mmol) Disuccinimidylcarbonat versetzt. Nach drei Stunden addiert man 0,965 g (10,6 mmol) 2,3-Dihydroxypropylamin. Man rührt über Nacht bei Raumtemperatur, dampft den Ansatz zur Trockne ein und rührt den Rückstand mit Diethylether aus. Der Feststoff wird abgesaugt und zur Reinigung an RP-Material chromatographiert.
Ausbeute: 0,82 g (76 % d. Th.)

| Analyse (bezogen auf lösungsmittelfreie Substanz): | | | | | |
|---|---|---|---|---|---|
| ber.: | C 55,32 | H 6,84 | N 6,79 | S 7,77 | O 23,27 |
| gef.: | C 55,39 | H 6,95 | N 6,57 | S 7,58 | |

### b) 4-[2-[4-Chlor-7-[5-N-(dihydroxypropyl)aminocarbonyl-3,3-dimethyl-1-(4-sulfonatobutyl)indolin-2-yliden]-3,5-(propan-1,3-diyl)-1,3,5-heptatrien-1-yl]-5-N-(dihydroxypropyl)aminocarbonyl-3,3-dimethyl(3H)indolio]butansulfonat, Natriumsalz

Eine Lösung von 360 mg (1 mmol) N-[5-Anilino-3-chlor-2,4-(propan-1,3-diyl)-2,4-pentadien-1-yliden]anilinium-chlorid, 825 mg (2 mmol) 5-N-(2,3-Dihydroxypropyl)aminocarbonyl-1-(4-sulfobutyl)-2,3,3-trimethyl(3H)indolenin (Beispiel 44a) und 330 mg (4 mmol) wasserfreies Natriumacetat in 30 ml Ethanol werden für zwei Stunden unter Argon am Rückfluß gekocht. Anschließend destilliert man das Ethanol ab und reinigt den Rückstand chromatographisch.
Ausbeute: 0,58 g (59 % d. Th.)

| Analyse (bezogen auf lösungsmittelfreie Substanz): | | | | | | | |
|---|---|---|---|---|---|---|---|
| ber.: | C 56,17 | H 6,15 | Cl 3,60 | N 6,79 | S 7,77 | O 23,27 | Na 2,34 |
| gef.: | C 55,99 | H 6,30 | Cl 3,41 | N 6,87 | S 7,64 | | Na 2,17 |

### c) 4-[2-[4-(4-(2-Carboxyethyl)phenyloxy)-7-[5-N-(dihydroxypropyl)aminocarbonyl-3,3-dimethyl-1-(4-sulfonatobutyl)indolin-2-yliden]-3,5-(propan-1,3-diyl)-1,3,5-heptatrien-1-yl]-5-N-(dihydroxypropyl)aminocarbonyl-3,3-dimethyl(3H)indolio]butansulfonat, Natriumsalz, N-Hydroxysuccinimidester

225 mg (1,4 mmol) 3-(4-Hydroxyphenyl)propionsäure werden in 10 ml trockenem N,N-Dimethylformamid unter Schutzgas gelöst und mit 65 mg (2,7 mmol) Natriumhydrid (60%-ig in Öl) versetzt. Nach 30 Minuten addiert man 138 mg (0,14 mmol) 4-[2-[4-Chlor-7-[5-N-(dihydroxypropyl)aminocarbonyl-3,3-dimethyl-1-(4-sulfonatobutyl)indolin-2-yliden]-3,5-(propan-1,3-diyl)-1,3,5-heptatrien-1-yl]-5-N-(dihydroxypropyl)aminocarbonyl-3,3-dimethyl(3H)indolio]butansulfonat, Natriumsalz (Beispiel 44b) und rührt für weitere 30 Minuten. Anschließend wird die Reaktionsmischung mit Trockeneis gequencht und zur Trockne eingedampft. Der Rückstand wird über eine präparative HPLC gereinigt. Zur Herstellung des Aktivesters löst man 14 mg (120 µmol) N-Hydroxysuccinimid und 2 mg (2,4 µmol) der Carbonsäure in 200 µl N,N-Dimethylformamid. Nach zehn Minuten setzt man 24 mg (120 µmol) Dicyclohexylcarbodiimid zu und rührt über Nacht bei Raumtemperatur. Der Aktivester wird ohne weitere Reinigung in die nächste Stufe eingesetzt.

### d) 4-[2-[4-(4-(2-Isothiocyanatoethyl)phenyloxy)-7-[5-N-(dihydroxypropyl)aminocarbonyl-3,3-dimethyl-1-(4-sulfonatobutyl)indolin-2-yliden]-3,5-(propan-1,3-diyl)-1,3,5-heptatrien-1-yl]-5-N-(dihydroxypropyl)aminocarbonyl-3,3-dimethyl(3H)indolio]butansulfonat, Natriumsalz

Zu einer Suspension von 28 mg (0,6 mmol) Natriumhydrid (60%-ig in Ö1) in 4 ml wasserfreiem N,N-Dimethylformamid addiert man bei 0°C 116 mg (0,6 mmol) 2-(4-Hydroxyphenyl)ethylisothiocyanat in 2 ml N,N-Dimethylformamid. Nach 30 Minuten wird die so hergestellte Lösung zu 138 mg (0,14 mmol) 4-[2-[4-Chlor-7-[5-N-(dihydroxypropyl)aminocarbonyl-5-N-(dihydroxypropyl)aminocarbonyl-3,3-dimethyl-1-(4-sulfonatobutyl)indolin-2-yliden]-3,5-(propan-1,3-diyl)-1,3,5-heptatrien-1-yl]-3,3-dimethyl(3H)indolio]butansulfonat, Natriumsalz (Beispiel 44b) gegeben. Man rührt über Nacht bei Raumtemperatur und quencht anschließend mit Trockeneis. Man dampft am Rotationsverdampfer zur Trockne ein und reinigt den Rückstand per präparativer HPLC.
Ausbeute: 85 mg (54 % d. Th.)

| Analyse (bezogen auf lösungsmittelfreie Substanz): | | | | | | |
|---|---|---|---|---|---|---|
| ber.: | C 58,65 | H 6,09 | N 6,22 | S 8,54 | O 18,47 | Na 2,04 |
| gef.: | C 58,53 | H 6,17 | N 6,11 | S 8,63 | | Na 1,83 |

In analoger Weise können weitere symmetrische hydrophile Farbstoffe aus folgenden Bausteinen aufgebaut werden:
Hydrophile Indolenin-Derivate mit Hydroxyalkylsubstituenten: (hergestellt nach Beispiel 44a)
   a) 5-N-(2,3-Dihydroxypropyl)-N-methylaminocarbonyl-1-(4-sulfobutyl)-2,3,3-trimethyl(3H)indolenin
   b) 5-N-(Hydroxyethyl)-aminocarbonyl-1-(4-sulfobutyl)-2,3,3-trimethyl(3H)indolenin
   c) 5-N-(2,3-Dihydroxypropyl)-N-(hydroxyethyl)-aminocarbonyl-1-(4-sulfobutyl)-2,3,3-trimethyl(3H)indolenin
   d) 5-N,N-(bis-Hydroxyethyl)-aminocarbonyl-1-(4-sulfobutyl)-2,3,3-trimethyl(3H)indolenin
   e) 5-N-(2,3,4,5,6-Pentahydroxyhexyl)-aminocarbonyl-1-(4-sulfobutyl)-2,3,3-trimethyl(3H)indolenin
   f) 5-N-(1,3,4-Trihydroxybut-2-yl)-N-methylaminocarbonyl-1-(4-sulfobutyl)-2,3,3-trimethyl(3H)indolenin

### Beispiel 45

### a) 5-N-(2,3,4,5,6-Pentahydroxyhexyl)aminocarbonyl-1-(4-sulfobutyl)-2,3,3-trimethyl(3H)indolenin

0,6 g (1,8 mmol) 5-Carboxy-1-(4-sulfobutyl)-2,3,3-Trimethyl(3H)indolenin (Anal. Biochem. 217,197, 1994) werden in 20 ml absolutem N,N-Dimethylformamid und 2 ml Pyridin vorgelegt und mit 0,95 g (3,6 mmol) Disuccinimidylcarbonat versetzt. Nach zwei Stunden addiert man 1,4 ml (10 mmol) Triethylamin und 322 mg (1,8 mmol) Glucamin. Man rührt über Nacht bei Raumtemperatur, dampft den Ansatz zur Trockne ein und rührt den Rückstand mit Diethylether aus. Der Feststoff wird abgesaugt und zur Reinigung an RP-Material chromatographiert.
Ausbeute: 0,67 g (74 % d. Th.)

| Analyse (bezogen auf lösungsmittelfreie Substanz): | | | | | |
|---|---|---|---|---|---|
| ber.: | C 52,58 | H 6,82 | N 5,57 | S 6,38 | O 28,65 |
| gef.: | C 52,47 | H 6,91 | N 5,39 | S 6,44 | |

### b) 4-[2-[4-Chlor-7-[5-N-(2,3,4,5,6-pentahydroxyhexyl)aminocarbonyl-3,3-dimethyl-1-(4-sulfonatobutyl)indolin-2-yliden]-3,5-(propan-1,3-diyl)-1,3,5-heptatrien-1-yl]-3,3-dimethyl5-[N-(2,3,4,5,6-pentahydroxyhexyl)aminocarbonyl] (3H)indolio]butansulfonat, Natriumsalz

Eine Lösung von 180 mg (0,5 mmol) N-[5-Anilino-3-chlor-2,4-(propan-1,3-diyl)-2,4-pentadien-1-yliden]anilinium-chlorid, 503 mg (1 mmol) 5-N-(2,3,4,5,6-Pentahydroxyhexyl)aminocarbonyl-1-(4-sulfobutyl)-2,3,3-trimethyl(3H)indolenin (Beispiel 45a) und 165 mg (2 mmol) wasserfreies Natriumacetat in 10 ml Ethanol werden für zwei Stunden unter Argon am Rückfluß gekocht. Anschließend destilliert man das Ethanol ab und reinigt den Rückstand chromatographisch.
Ausbeute: 0,31 g (53 % d. Th.)

| Analyse (bezogen auf lösungsmittelfreie Substanz): | | | | | | | |
|---|---|---|---|---|---|---|---|
| ber.:C | 54,05 | H 6,33 | Cl 3,01 | N 4,76 | S 5,44 | O 24,45 | Na 1,95 |
| gef.:C | 53,89 | H 6,20 | Cl 2,87 | N 4,83 | S 5,29 | | Na 1,72 |

### c) 4-[2-[4-(4-Isothiocyanatothiophenyloxy)-7-[5-N-(2,3,4,5,6-pentahydroxyhexyl)aminocarbonyl-3,3-dimethyl-1-(4-sulfonatobutyl)indolin-2-yliden]-3,5-(propan-1,3-diyl)-1,3,5-heptatrien-1-yl]-3,3-dimethyl-5-[N-(2,3,4,5,6-pentahydroxyhexyl)aminocarbonyl](3H)indolio]butansulfonat, Natriumsalz

54 mg (0,4 mmol) 4-Aminothiophenol werden in 10 ml absolutem N,N-Dimethylformamid unter Argonatmosphäre gelöst und bei Raumtemperatur mit 165 mg (0,14 mmol) 4-[2-[4-Chlor-7-[5-N-(2,3,4,5,6-pentahydroxyhexyl)aminocarbonyl-3,3-dimethyl-1-(4-sulfonatobutyl)indolin-2-yliden]-3,5-(propan-1,3-diyl)-1,3,5-heptatrien-1-yl]-3,3-dimethyl-5-[N-(2,3,4,5,6-pentahydroxyhexyl)aminocarbonyl](3H)indolio]butansulfonat, Natriumsalz (Beispiel 45b) versetzt. Nach 10 Minuten wird die Reaktionsmischung mit Trockeneis gequencht und 210 mg (1 mmol) Thiocarbonyldiimidazol addiert. Nach 45 Minuten wird der Farbstoff mit Diethylether gefällt und der Feststoff per Zentrifugation isoliert. Zur Reinigung kann an RP-Material chromatographiert werden.
Ausbeute: 78 mg (43 % d. Th.)

| Analyse (bezogen auf lösungsmittelfreie Substanz): | | | | | | |
|---|---|---|---|---|---|---|
| ber.: | C 55,07 | H 6,01 | N 5,35 | S 9,80 | O 22,01 | Na 1,76 |
| gef.: | C 54,89 | H 6,20 | N 5,24 | S 9,58 | | Na 1,54 |

In analoger Weise können weitere symmetrische hydrophile Farbstoffe aus folgenden Bausteinen aufgebaut werden:
Hydrophile Indolenin-Derivate mit Hydroxyalkylsubstituenten: (hergestellt nach Beispiel 44a)
   a) 5-N-(2,3-Dihydroxypropyl)-N-methylaminocarbonyl-1-(4-sulfobutyl)-2,3,3-trimethyl(3H)indolenin
   b) 5-N-(Hydroxyethyl)-aminocarbonyl-1-(4-sulfobutyl)-2,3,3-trimethyl(3H)indolenin
   c) 5-N-(2,3-Dihydroxypropyl)-N-(hydroxyethyl)-aminocarbonyl-1-(4-sulfobutyl)-2,3,3-trimethyl(3H)indolenin
   d) 5-N,N-(bis-Hydroxyethyl)-aminocarbonyl-1-(4-sulfobutyl)-2,3,3-trimethyl(3H)indolenin
   e) 5-N-(2,3,4,5,6-Pentahydroxyhexyl)-aminocarbonyl-1-(4-sulfobutyl)-2,3,3-trimethyl(3H)indolenin
   f) 5-N-(1,3,4-Trihydroxybut-2-yl)-N-methylaminocarbonyl-1-(4-sulfobutyl)-2,3,3-trimethyl(3H)indolenin

### Beispiel 46

### 7-[5-N-(2,3-Dihydroxypropyl)aminocarbonyl-3,3-dimethyl-1-(4-sulfonatobutyl)indolin-2-yliden]-1,3,5-heptatrien-1-yl]-3,3-dimethyl-5-carboxy(3H)indolio]butansulfonat, Natriumsalz

Eine Lösung von 2,35 g (5,71 mmol) 5-N-(2,3-Dihydroxypropyl)aminocarbonyl-1-(4-sulfobutyl)-2,3,3-trimethyl(3H)indolenin (Beispiel 44a) und 1,57 g (5,5 mmol) Glutaconaldehyddianilid-hydrochlorid in 25 ml Essigsäureanhydrid werden 30 Minuten bei 120°C gerührt. Anschließend addiert man 2,4 g (7,1 mmol) 5-Carboxy-1-(4-sulfobutyl)-2,3,3-trimethyl(3H)indolenin, 1,71 g Natriumacetat, 22 ml Essigsäureanhydrid und 8,6 ml Essigsäure. Man läßt die Reaktionsmischung eine Stunde bei 120°C rühren, kühlt auf Raumtemperatur ab und fällt das Produkt mit Diethylether. Das Rohprodukt wird über RP-Material chromatographiert.
Ausbeute: 1,9 g (40 % d. Th.)

| Analyse (bezogen auf lösungsmittelfreie Substanz): | | | | | | |
|---|---|---|---|---|---|---|
| ber.: | C 54,47 | H 6,03 | N 5,03 | S 7,67 | O 21,05 | Na 2,75 |
| gef.: | C 54,26 | H 6,12 | N 5,00 | S 7,49 | | Na 2,48 |

Analog zur Synthese nach Beispiel 14-16 und 18-27 a (Festphasenpeptidsynthese) wird das VIP-Analogon HSDAVFTDNY TRLRKKMAVK KYLNSILN an der Festphase synthetisiert. Der Farbstoff 7-[5-N-(2,3-Dihydroxypropyl)aminocarbonyl-3,3-dimethyl-1-(4-sulfonatobutyl)indolin-2-yliden]-1,3,5-heptatrien-1-yl]-3,3-dimethyl-5-carboxy(3H)indolio]butansulfonat, Natriumsalz wird nach Beispiel 14-16 und 18-27 b (Farbstoffkopplung) N-terminal an das Peptid gekoppelt und nach Beispiel 14-16 und 18-27 c (Schutzgruppenabspaltung und Ablösung der Farbstoff-Peptid-Konjugate) wird dieses Konjugat isoliert und mittels HPLC gereinigt.

In analoger Weise können weitere unsymmetrische hydrophile Farbstoffe aus folgenden Bausteinen aufgebaut werden:
Hydrophile Indolenin-Derivate mit Hydroxyalkylsubstituenten: (hergestellt nach Beispiel 44a)
   a) 5-N-(2,3-Dihydroxypropyl)-N-methylaminocarbonyl-1-(4-sulfobutyl)-2,3,3-trimethyl(3H)indolenin
   b) 5-N-(Hydroxyethyl)-aminocarbonyl-1-(4-sulfobutyl)-2,3,3-trimethyl(3H)indolenin
   c) 5-N-(2,3-Dihydroxypropyl)-N-(hydroxyethyl)-aminocarbonyl-1-(4-sulfobutyl)-2,3,3-trimethyl(3H)indolenin
   d) 5-N,N-(bis-Hydroxyethyl)-aminocarbonyl-1-(4-sulfobutyl)-2,3,3-trimethyl(3H)indolenin
   e) 5-N-(2,3,4,5,6-Pentahydroxyhexyl)-aminocarbonyl-1-(4-sulfobutyl)-2,3,3-trimethyl(3H)indolenin
   f) 5-N-(1,3,4-Trihydroxybut-2-yl)-N-methylaminocarbonyl-1-(4-sulfobutyl)-2,3,3-trimethyl(3H)indolenin
Indoleninderivate mit Carboxylgruppe:
   a) 5-Carboxy-1-(4-sulfobutyl)-2,3,3-trimethyl(3H)indolenin
   b) 5-Carboxymethyl-1-(4-sulfobutyl)-2,3,3-trimethyl(3H)indolenin
   c) 5-Carboxy-1-(3-sulfopropyl)-2,3,3-trimethyl(3H)indolenin
   d) 5-Carboxymethyl-1-(3-sulfopropyl)-2,3,3-trimethyl(3H)indolenin
   e) 5-Carboxy-1-(2-sulfoethyl)-2,3,3-trimethyl(3H)indolenin
   f) 5-Carboxymethyl-1-(2-sulfoethyl)-2,3,3-trimethyl(3H)indolenin
Dianilderivate zur Reaktion mit o. g. Indoleninen unter Bildung von Mono-, Di- oder Tricarbocyaninen:
   a) Glutaconaldehyddianilid-hydrochlorid
   b) Malonaldehyd-bis-phenylimin-hydrochlorid
   c) N,N-Diphenylformamidin
   d) N-[5-Anilino-2,4-(propan-1,3-diyl)-2,4-pentadien-1-yliden]anilinium-chlorid
   e) N-[5-Anilino-2,4-(ethan-1,2-diyl)-2,4-pentadien-1-yliden]anilinium-chlorid
   f) N-[5-Anilino-3-chlor-2,4-(propan-1,3-diyl)-2,4-pentadien-1-yliden]anilinium-chlorid
   g) N-[5-Anilino-3-chlor-2,4-(ethan-1,2-diyl)-2,4-pentadien-1-yliden]anilinium-chlorid

### Beispiel 47

### 7-[5-N-(1,3,4-Trihydroxybut-2-yl)-aminocarbonyl-3,3-dimethyl-1-(4-sulfonatobutyl)indolin-2-yliden]- 3,5-(2-carboxypropan-1,3-diyl)-1,3,5-heptatrien-1-yl]-3,3-dimethyl-5- N-(1,3,4-trihydroxybut-2-yl)-aminocarbonyl(3H)indolio]butansulfonat, Natriumsalz, N-Hydroxysuccinimidester

### a) 4-[2-[4-Chlor-7-[5-N-(1,3,4-trihydroxybut-2-yl)aminocarbonyl-3,3-dimethyl-1-(4-sulfonatobutyl)indolin-2-yliden]-3,5-[2-(methoxycarbonyl)propan-1,3-diyl]-1,3,5-heptatrien-1-yl]-3,3-dimethyl-5-[N-(1,3,4-trihydroxybut-2-yl)aminocarbonyl] (3H)indolio]butansulfonat, Natriumsalz

Zu einer Lösung von 0,8 g (5 mmol) 4-(Methoxycarbonyl)-cyclohexanon in 5 ml Dichlormethan addiert man 5,5 ml (10 mmol) Phosphoroxychlorid und 6 ml N,N-Dimethylformamid bei 0°C. Anschließend erhitzt man eine Stunde am Rückfluß. Man destilliert das Dichlormethan ab und fügt bei maximal 5°C 4 ml Anilin in 10 ml Methanol hinzu. Man gießt die Reaktionsmischung auf Eis, gibt 5 ml konzentrierte Salzsäure hinzu und läßt das Zwischenprodukt fünf Stunden bei 0°C auskristallisieren. Man saugt die Kristalle ab und setzt diese ohne weitere Reinigung in die nächste Reaktion ein. Dazu löst man die Kristalle in wasserfreiem Ethanol und fügt 4,4 g (10 mmol) 5-N-(1,3,4-Trihydroxybut-2-yl)-N-methylaminocarbonyl-1-(4-sulfobutyl)-2,3,3-trimethyl(3H)indolenin (s. Beispiel 45) und 0,8 g wasserfreies Natriumacetat hinzu. Man erhitzt für eine Stunde am Rückfluß, filtriert den Feststoff ab und dampft das Filtrat zur Trockne ein. Der Rückstand wird zur Reinigung chromatographiert.
Ausbeute: 3,25 g (58 % d. Th.)

| Analyse (bezogen auf lösungsmittelfreie Substanz): | | | | | | | |
|---|---|---|---|---|---|---|---|
| ber.: | C 54,90 | H 6,14 | Cl 3,18 | N 5,02 | S 5,75 | O 22,94 | Na 2,06 |
| gef.: | C 54,76 | H 6,03 | Cl 2,99 | N 4,91 | S 5,60 | | Na 1,83 |

### b) 7-[5-N-(1,3,4-Trihydroxybut-2-yl)aminocarbonyl-3,3-dimethyl-1-(4-sulfonatobutyl)indolin-2-yliden]- 3,5-(2-carboxypropan-1,3-diyl)-1,3,5-heptatrien-1-yl]-3,3-dimethyl-5- N-(1,3,4-trihydroxybut-2-yl)aminocarbonyl(3H)indolio]butansulfonat, Natriumsalz

Eine Mischung von 10 mg (0,4 mmol) Natriumhydrid und 80 mg (1,3 mmol) Ethanthiol in 10 ml wasserfreiem N,N-Dimethylformamid werden unter Stickstoff 30 Minuten bei Raumtemperatur gerührt. Anschließend versetzt man mit 112 mg (0,1 mmol) 4-[2-[4-Chlor-7-[5-N-(2,3,4,5,6-pentahydroxyhexyl)aminocarbonyl-3,3-dimethyl-1-(4-sulfonatobutyl)indolin-2-yliden]-3,5-[2-(methoxycarbonyl)propan-1,3-diyl]-1,3,5-heptatrien-1-yl]-3,3-dimethyl5-[N-(2,3,4,5,6-pentahydroxyhexyl)-aminocarbonyl] (3H)indolio]butansulfonat, Natriumsalz (Beispiel 47a) in 3 ml N,-N-Dimethylformamid. Die Reaktionsmischung wird für zwei Stunden auf 100°C erhitzt und nach Abkühlung auf Raumtemperatur mit Kohlendioxid gequencht. Man dampft am Rotationsverdampfer zur Trockne ein und extrahiert den Rückstand mit heißem Ethanol. Man dampft den Extrakt ein und chromatographiert das Rohprodukt.
Ausbeute: 75 mg (67 % d. Th.)

| Analyse (bezogen auf lösungsmittelfreie Substanz): | | | | | | |
|---|---|---|---|---|---|---|
| ber.: | C 56,27 | H 6,33 | N 5,25 | S 6,01 | O 23,99 | Na 2,15 |
| gef.: | C 56,13 | H 6,46 | N 5,12 | S 5,87 | | Na 1,88 |

### c) 7-[5-N-(1,3,4-Trihydroxybut-2-yl)-aminocarbonyl-3,3-dimethyl-1-(4-sulfonatobutyl)indolin-2-yliden]- 3,5-(2-carboxypropan-1,3-diyl)-1,3,5-heptatrien-1-yl]-3,3-dimethyl-5- N-(1,3,4-trihydroxybut-2-yl)-aminocarbonyl(3H)indolio]butansulfonat-N-Hydroxysuccinimidester, Natriumsalz

Zur Herstellung des Aktivesters löst man 14 mg (0,12 mmol) N-Hydroxysuccinimid und 64 mg (0,06 mmol) der Carbonsäure in 2 ml N,N-Dimethylformamid. Nach 15 Minuten setzt man 25 mg (0,12 mmol) Dicyclohexylcarbodiimid zu und rührt über Nacht bei Raumtemperatur. Der Aktivester wird mittels präparativer HPLC gereinigt.
Ausbeute: 60 mg (86 % d. Th.)

| Analyse (bezogen auf lösungsmittelfreie Substanz): | | | | | | |
|---|---|---|---|---|---|---|
| ber.: | C 55,71 | H 6,06 | N 6,02 | S 5,51 | O 24,73 | Na 1,97 |
| gef.: | C 55,59 | H 6,21 | N 5,93 | S 5,37 | | Na 1,75 |

### Beispiel 48

### 7-[5-N-(1,3,4-Trihydroxybut-2-yl)-aminocarbonyl-3,3-dimethyl-1-(4-sulfonatobutyl)indolin-2-yliden]- 3,5-(2-carboxypropan-1,3-diyl)-1,3,5-heptatrien-1-yl]-3,3-dimethyl-5- N-(1,3,4-trihydroxybut-2-yl)-aminocarbonyl(3H)indolio]butansulfonat, Natriumsalz

### a) 5-N-(11-Aminoundecyl)aminocarbonyl-1-(4-sulfobutyl)-2,3,3-trimethyl(3H)indolenin

340 mg (1 mmol) 5-Carboxy-1-(4-sulfobutyl)-2,3,3-trimethyl(3H)indolenin (Anal. Biochem. 217,197, 1994) werden in 5 ml absolutem N,N-Dimethylformamid und 1 ml Pyridin vorgelegt und mit 0,5 g (2 mmol) Disuccinimidylcarbonat versetzt. Nach drei Stunden addiert man 0,805 g (4 mmol) 11-Aminoundecansäure. Man rührt über Nacht bei Raumtemperatur, dampft den Ansatz zur Trockne ein und rührt den Rückstand mit Diethylether aus. Der Feststoff wird abgesaugt und zur Reinigung an RP-Material chromatographiert.
Ausbeute: 0,37 g (71 % d. Th.)

| Analyse (bezogen auf lösungsmittelfreie Substanz): | | | | | |
|---|---|---|---|---|---|
| ber.: | C 62,04 | H 8,10 | N 5,36 | S 6,13 | O 18,37 |
| gef.: | C 61,88 | H 8,23 | N 5,17 | S 6,02 | |

### b) 7-[5-N-(1,3,4-Trihydroxybut-2-yl)-aminocarbonyl-3,3-dimethyl-1-(4-sulfonatobutyl)indolin-2-yliden]- 3,5-(2-carboxypropan-1,3-diyl)-1,3,5-heptatrien-1-yl]-3,3-dimethyl-5- N-(1,3,4-trihydroxybut-2-yl)-aminocarbonyl(3H)indolio]butansulfonat, Natriumsalz

Eine Lösung von 0,35 g (0,67 mmol) 5-N-(11-Aminoundecyl)aminocarbonyl-1-(4-sulfobutyl)-2,3,3-trimethyl(3H)indolenin (Beispiel 48a) und 0,18 g (0,645 mmol) Glutaconaldehyddianil in 3 ml Essigsäureanhydrid werden 20 Minuten bei 110°C gerührt. Anschließend addiert man 344 mg (0,83 mmol) 5-N-(2,3-Dihydroxypropyl)-aminocarbonyl-1-(4-sulfobutyl)-2,3,3-trimethyl(3H-)indolenin (Beispiel 44a), 0,2 g Natriumacetat, 3 ml Essigsäureanhydrid und 1 ml Essigsäure. Man läßt die Reaktionsmischung zwei Stunden bei 110°C rühren, kühlt auf Raumtemperatur ab und fällt das Produkt mit Diethylether. Das Rohprodukt wird über RP-Material chromatographiert. Ausbeute: 0,41 g (60 % d. Th.)

| Analyse (bezogen auf lösungsmittelfreie Substanz): | | | | | | |
|---|---|---|---|---|---|---|
| ber.: | C 60,10 | H 7,02 | N 5,50 | S 6,29 | O 18,84 | Na 2,26 |
| gef.: | C 59,96 | H 7,14 | N 5,33 | S 6,15 | | Na 2,11 |

Weitere erfindungsgemäße Farbstoffe können nach Beispiel 44a und b hergestellt werden, in dem man an Stelle von 11-Aminoundecansäure in Beispiel 48a folgende Aminosäuren und die in Beispiel 46 genannten Indoleninderivate mit Hydroxyalkylsubstituenten a)-f) verwendet:
i. Glycin
ii. Alanin
iii. β-Alanin
iv. 4-Aminobutansäure
v. 6-Aminohexansäure
vi. H₂N-(CH₂CH₂O)₃CH₂COOH (TH 53, 20, 6977)
vii. H₂N-(CH₂CH₂O)₄CH₂COOH (JOC 63, 5, 1728, 1998)
viii. H₂N-CH₂CH₂COO(CH₂CH₂O)₄-CO-CH₂CH₂COOH (Lett. Pept. Sci. 6, 135, 1999)
ix. HCl* H₂N-PEG-COOH (MW 3400 g/mol; Shearwater Polymers Inc., USA)

### Beispiel 49

### 4-[2-[4-(4-(N-(4-Aza-6-brom-5-oxohexyl)aminocarbonyl-ethyl)phenyloxy)-7-[5-N-(dihydroxypropyl)aminocarbonyl-3,3-dimethyl-1-(4-sulfonatobutyl)indolin-2-yliden]-3,5-(propan-1,3-diyl)-1,3,5-heptatrien-1-yl]-5-N-(dihydroxypropyl)aminocarbonyl-3,3-dimethyl(3H)indolio]butansulfonat, Natriumsalz

### a) [3-[N-(tert.-Butoxycarbonyl)amino]propyl]-N'-(bromacetyl)-amid

2,5 g (14,4 mmol) [3-[N-(tert.-Butoxycarbonyl)amino]-propyl]amin werden in 15 ml Dioxan gelöst und nach Zugabe von 4,4 ml Triethylamin bei 0°C mit 3,2 g (16 mmol) Bromacetylbromid versetzt. Man rührt über Nacht bei Raumtemperatur und addiert anschließend weitere 320 mg Bromacetylbromid. Nach zwei Stunden bei Raumtemperatur wird der Niederschlag abgesaugt, die Lösung eingeengt und der Rückstand in Essigsäureethylester aufgenommen. Man wäscht mit Wasser und trocknet die organische Phase über Natriumsulfat.
Ausbeute: 3,2 g (75.% d. Th.)

| Analyse (bezogen auf lösungsmittelfreie Substanz): | | | | | |
|---|---|---|---|---|---|
| ber.: | C 40,69 | H 6,49 | Br 27,07 | N 9,49 | O 16,26 |
| gef.: | C 40,50 | H 6,37 | Br 26,89 | N 9,58 | |

### b) [3-[N-( Bromacetyl)amino]propyl]amin, Hydrochlorid

3,1 g (10,5 mmol) [3-[N-(tert.-Butoxycarbonyl)amino-]propyl]-N'-(bromacetyl)-amid (Beispiel 49a) werden mit 50 mmol 1M Salzsäure in Essigsäureethylester für fünf Stunden bei Raumtemperatur gerührt. Man saugt das Produkt ab und wäscht den Feststoff mit Essigsäureethylester nach.
Ausbeute: 2,3 g (95 % d. Th.)

| Analyse (bezogen auf lösungsmittelfreie Substanz): | | | | | | |
|---|---|---|---|---|---|---|
| ber.: | C 25,94 | H 5,22 | Cl 15,31 | Br 34,51 | N 12,10 | O 6,91 |
| gef.: | C 25,76 | H 5,41 | Cl 15,55 | Br 34,34 | N 11,97 | |

### c) 4-[2-[4-(4-(N-(4-Aza-6-brom-5-oxo-hexyl)aminocarbonyl-ethyl)phenyloxy)-7-[5-N-(dihydroxypropyl)aminocarbonyl-3,3-dimethyl-1-(4-sulfonatobutyl)indolin-2-yliden]-3,5-(propan-1,3-diyl)-1,3,5-heptatrien-1-yl]-5-N-(dihydroxypropyl)aminocarbonyl-3,3-dimethyl(3H)indolio]butansulfonat, Natriumsalz

121 mg (0,1 mmol) 4-[2-[4-(4-(2-Carboxyethyl)phenyloxy)-7-[5-N-(dihydroxypropyl)aminocarbonyl-3,3-dimethyl-1-(4-sulfonatobutyl)indolin-2-yliden]-3,5-(propan-1,3-diyl)-1,3,5-heptatrien-1-yl]-5-N-(dihydroxypropyl)-aminocarbonyl-3,3-dimethyl(3H)indolio]butansulfonat, Natriumsalz, N-Hydroxysuccinimidester (Beispiel 44c) werden in 0,5 ml N,N-Dimethylformamid gelöst, mit 0,06 ml Triethylamin und 70 mg (0,3 mmol) [3-[N-( Bromacetyl)amino]propyl]amin, Hydrochlorid (Beispiel 49b) versetzt.
Man rührt vier Stunden bei 60°C, kühlt anschließend auf Raumtemperatur ab und fällt das Produkt mit Diethylether. Der Feststoff wird abgesaugt und mit reichlich Diethylether gewaschen.
Ausbeute: 0,11 mg (80 % d. Th.)

| Analyse (bezogen auf lösungsmittelfreie Substanz): | | | | | | | |
|---|---|---|---|---|---|---|---|
| ber.: | C 56,17 | H 6,18 | Br 6,13 | N 6,44 | S 4,92 | Na 1,76 | O 18,40 |
| gef.: | C 55,96 | H 6,26 | Br 6,01 | N 6,27 | S 4,81 | Na 1,53 | |

## Patentansprüche

1. Peptid-Polymethinfarbstoff-Konjugate der allgemeinen Formel (I)
**A¹ - (X)ₘ - A²** **I**
worin
X für eine α, β oder γ-Aminosäure mit D oder L-Konfiguration und
m für eine Zahl von 5 bis 30 steht,
wobei die resultierende Aminosäuresequenz (X)ₘ geradkettiger Natur oder über eine Disulfidbrücke zwischen zwei Cysteinen oder Homocysteinen oder amidisch zwischen N- und C-Terminus cyclisiert sein kann und für die Aminosäuresequenz des Somatostatins oder für Fragmente, Teilsequenzen, Derivate oder Analoga des Somatostatins steht,
A¹ für ein Wasserstoffatom, einen Acetylrest oder einen Alkylrest mit bis zu 10 C-Atomen, der gegebenenfalls mit 1 bis 3 Carboxygruppen und/oder 1 bis 6 Hydroxygruppen substituiert sein kann, oder einen Poly(oxyethylen)rest mit 2 bis 30 -CH₂CH₂O-Einheiten oder ein Farbstoffmolekül aus der Klasse der Polymethinfarbstoffe, welches mindestens ein Absorptionsmaximum im Bereich von 380 bis 1200 nm aufweist, steht
A² für eine Hydroxygruppe, eine Aminogruppe oder ein Farbstoffmolekül aus der Klasse der Polymethinfarbstoffe, welches mindestens ein Absorptionsmaximum im Bereich von 380 bis 1200 nm aufweist, steht
unter der Bedingung, daß mindestens einer der Reste A¹ oder A² ein Farbstoffmolekül aus der Klasse der Polymethinfarbstoffe, welches mindestens ein Absorptionsmaximum im Bereich von 380 bis 1200 nm aufweist, darstellt,
wobei für den Fall, daß A¹ und/oder A² ein Farbstoffmolekül aus der Klasse der Polymethinfarbstoffe, welches mindestens ein Absorptionsmaximum im Bereich von 380 bis 1200 nm aufweist, darstellen, A¹ an die N-terminale Aminogruppe und A² an eine Aminogruppe der Aminosäure Lysin oder an eine Hydroxygruppe der Aminosäure Serin in beliebiger Position innerhalb der Aminosäuresequenz (X)ₘ geknüpft ist,
wobei Farbstoffmolekül aus der Klasse der Polymethinfarbstoffe für einen Cyaninfarbstoff der allgemeinen Formeln II, steht, 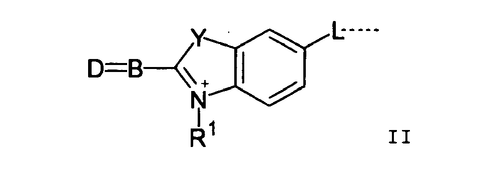 worin
D für ein Fragment entsprechend den allgemeinen Formeln III bis VI,wobei die mit einem Stern **gekennzeichnet**e Position die Verknüpfung mit B bedeutet, steht, 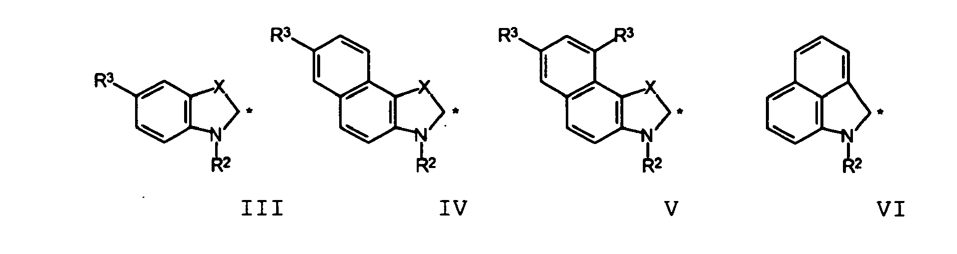 B für ein Fragment entsprechend den allgemeinen Formeln VII bis XII 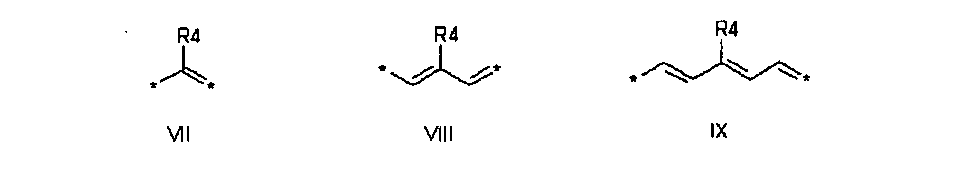 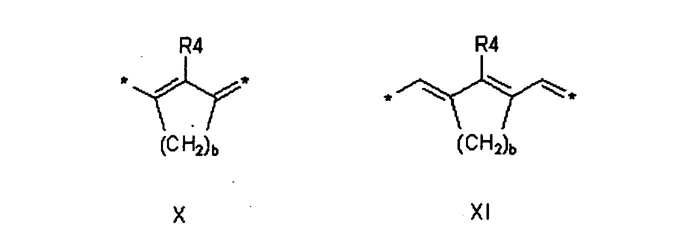 R¹ und R² für E¹, R³ für ein Fluor-, Chlor-, Brom-, Iodatom oder eine Nitrogruppe oder für einen Rest -COOE¹, - CONE¹E², -NHCOE¹, -NHCONHE¹, -NE¹E², -OE¹, -OSO₃E¹, - SO₃E¹, -SO₂NHE¹, -E¹,
wobei E¹ und E² unabhängig voneinander für ein Wasserstoffatom, eine C₁-C₄-Sulfoalkylkette, eine gesättigte oder ungesättigte, verzweigte oder geradkettige C₁-C₅₀-Alkylkette, wobei die Kette oder Teile dieser Kette gegebenenfalls eine oder mehrere aromatische oder gesättigte zyklische C₅-C₆- oder bizyklische C₁₀-Einheiten formen können, steht, und wobei die C₁-C₅₀-Alkylkette von 0 bis 15 Sauerstoffatomen und/oder von 0 bis 3 Carbonylgruppen unterbrochen ist und/oder mit 0 bis 5 Hydroxygruppen substituiert ist, stehen,
R⁴ für ein Wasserstoffatom, ein Fluor-, Chlor-, Brom-, Iodatom oder eine verzweigte oder geradkettige C₁-C₁₀-Alkylkette, steht,
b eine Zahl 2 oder 3 bedeutet,
X und Y unabhängig voneinander O, S, Se, -CH=CH- oder C(CH₃)₂
bedeuten,
L für eine Gruppe entsprechend nachstehender Formeln 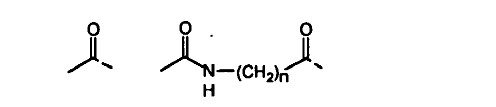 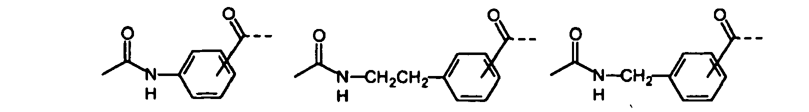
worin
n eine Zahl von 1 bis 10 bedeutet,
steht,
und deren physiologisch verträgliche Salze.

2. Verbindungen nach Anspruch 1, **dadurch gekennzeichnet, daß** das Farbstoffmolekül A¹ und/oder A² für einen Indocarbocyanin-, einen Indodicarbocyanin- oder einen Indotricarbocyaninfarbstoff steht.

3. Verbindungen nach Anspruch 2, **dadurch gekennzeichnet, daß** das Farbstoffmolekül A¹ und/oder A² für einen Indocarbocyanin-, einen Indodicarbocyanin- oder einen Indotricarbocyaninfarbstoff der allgemeinen Formel XIII oder XIV 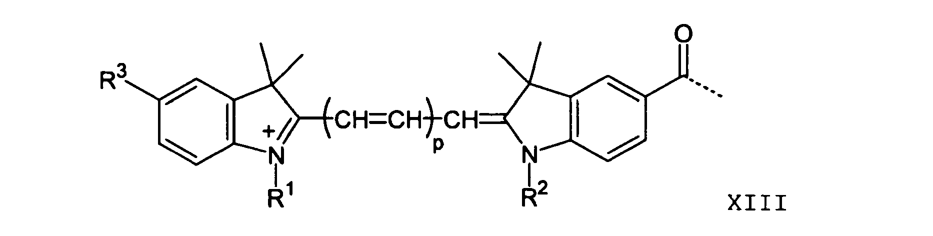 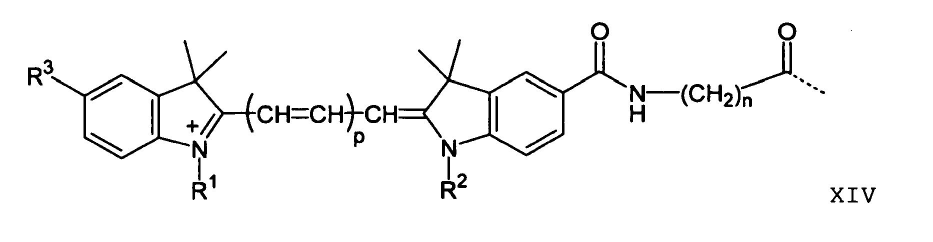 worin
p für 1, 2 oder 3,
n für 1, 2, 3, 4 oder 10 steht,
R¹ und R² unabhängig voneinander für einen 4-Sulfobutyl-, 3-Sulfopropyl-, 2-Sulfoethyl-, 3-Methyl-3-sulfopropyl-, Methyl-, Ethyl- oder Propylrest stehen, und
R³ für Wasserstoff, ein Chlor-, Brom-, Iodatom oder eine Nitrogruppe oder für einen Rest -COOE¹, -CONE¹E², -NHCOE¹, -NHCONHE¹, -NE¹E², -OE¹, -OSO₃E¹, -SO₃E¹, -SO₂NHE¹, wobei E¹ und E² unabhängig voneinander für ein Wasserstoffatom oder für einen Methyl-, Ethyl- oder einen C₃-C₆-Alkylrest, der von 0 bis 2 Sauerstoffatomen und/oder von 0 bis 1 Carbonylgruppen unterbrochen ist und/oder mit 0 bis 5 Hydroxygruppen substituiert ist, oder für einen Poly(oxyethylen)glykolrest mit 2 bis 30 -CH₂CH₂O-Einheiten stehen,
steht.

4. Verbindungen nach Anspruch 2, **dadurch gekennzeichnet, daß** das Farbstoffmolekül A¹ und/oder A² für einen Indocarbocyanin-, einen Indodicarbocyanin- oder einen Indotricarbocyaninfarbstoff der allgemeinen Formel XIII oder XIV 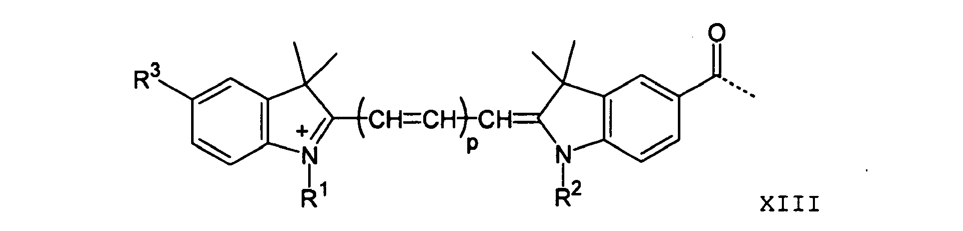 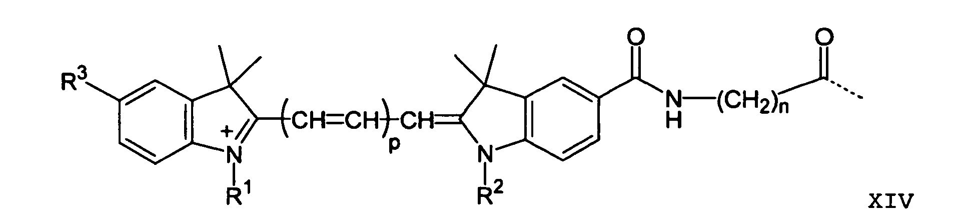 worin
p für 1, 2 oder 3,
n für 1, 2 oder 4,
R¹ und R² unabhängig voneinander für einen 4-Sulfobutyl-oder 3-Sulfopropylrest,
R³ für Wasserstoff oder für einen Rest -COOE¹ oder -CONHE¹,
wobei E¹ ein Wasserstoffatom oder ein Methyl-, Ethyl- oder ein C₃-C₆-Alkylrest, der von 0 bis 2 Sauerstoffatomen und/oder von 0 bis 1 Carbonylgruppen unterbrochen ist und/oder mit 0 bis 5 Hydroxygruppen substituiert ist, bedeutet,
steht.

5. Verbindungen nach Anspruch 2, **dadurch gekennzeichnet, daß** das Farbstoffmolekül A¹ und/oder A² für einen Indotricarbocyaninfarbstoff der allgemeinen Formel XV oder XVI steht: 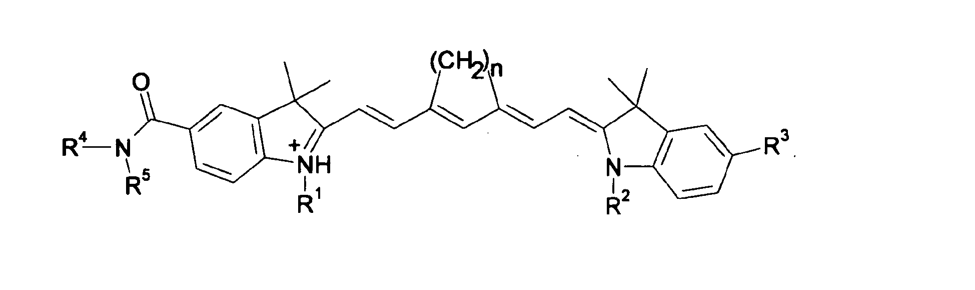 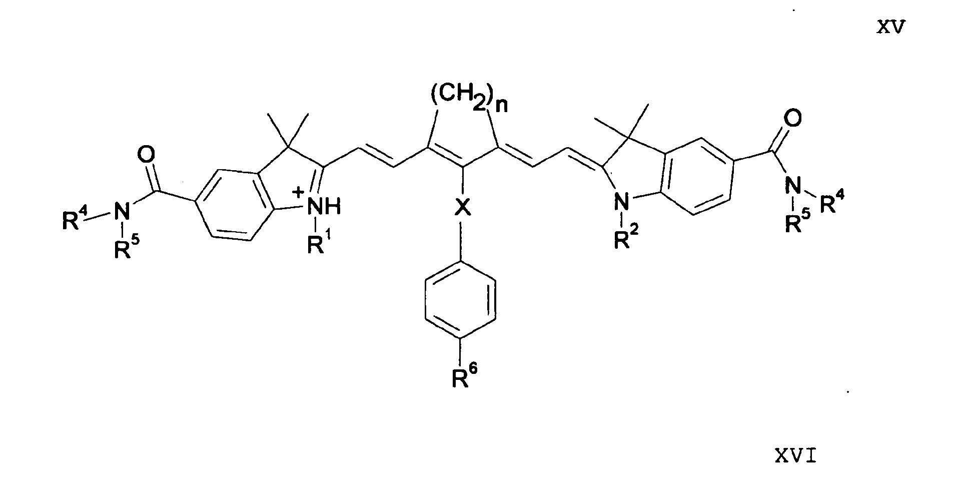 worin
n für 2 oder 3 steht,
R¹ und R² unabhängig voneinander einen 4-Sulfobutyl-, 3-Sulfopropyl- oder 2-Sulfoethylrest darstellen,
R³ für einen Rest -CONH-Peptid, -CONH-(CH₂)ₘ-CONH-Peptid, -CONH-(CH₂)ₙ-NH-CS-NH-Peptid oder -CONH-(CH₂)ₙ-NHCO-CH₂-Peptid mit m = 1 bis 10 und n = 2 oder 3 steht,
oder eine folgende Gruppe darstellt: 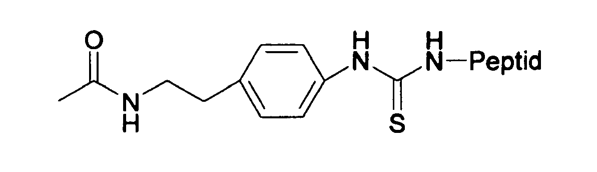 R⁴ und R⁵ unabhängig voneinander für ein Wasserstoffatom, einen Methylrest oder einen hydroxylierten Alkylrest stehen,
R⁶ für eine der folgenden Gruppen steht:
-(CH₂)ₘ-CONH-Peptid mit m = 0 bis 2,
-(CH₂)ₘ-NH-CS-NH-Peptid mit m = 0 bis 2,
und X für ein Sauerstoffatom oder ein Schwefelatom steht.

6. Verbindungen nach Anspruch 2, **dadurch gekennzeichnet, daß** das Farbstoffmolekül A¹ und/oder A² für einen Indotricarbocyaninfarbstoff der allgemeinen Formel XVII steht: 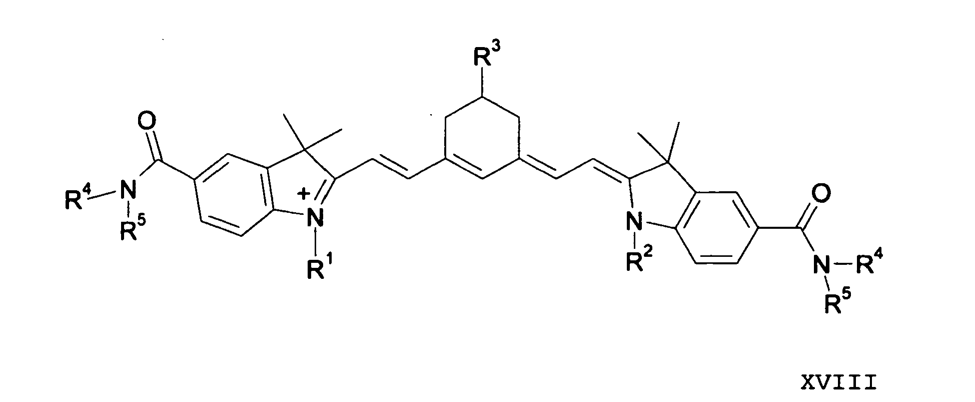 worin
R¹ und R² unabhängig voneinander für einen 4-Sulfobutyl, 3-Sulfopropyl- oder 2-Sulfoethylrest darstellen,
R³ für einen Rest -CONH-Peptid, -NH-CS-NH-Peptid oder -CONH-(CH₂)ₙ-NHCO-CH₂-Peptid mit n = 2 oder 3 steht oder eine folgende Gruppe darstellt: 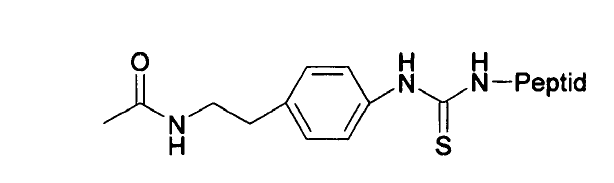 und R⁴ und R⁵ unabhängig voneinander für ein Wasserstoffatom, einen Methylrest oder einen hydroxylierten Alkylrest stehen.

7. Verbindungen gemäß Anspruch 5 oder 6, **dadurch gekennzeichnet, daß** die hydroxylierten Alkylreste für 2-Hydroxyethyl, 3-Hydroxypropyl, 2,3-Dihydroxypropyl, 1,3-Dihydroxy-2-propyl, 2,3,4-Trihydroxybutyl, 1,3,4-Trihydroxy-2-butyl, 2,3,4,5,6-Pentahydroxyhexyl stehen.

8. Verbindungen nach Anspruch 1, **dadurch gekennzeichnet, daß** (X)ₘ für die Aminosäuresequenz des Somatostatins entsprechend 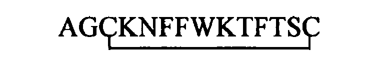 oder für Fragmente, Teilsequenzen, Derivate oder Analoga des Somatostatins, bestehend aus 5 bis 20 Aminosäuren, steht.

9. Verbindungen nach mindestens einem der vorhergehenden Ansprüche, **dadurch gekennzeichnet, daß** 2 bis m Aminosäuren unabhängig voneinander gegen ihre jeweilige D-Aminosäure oder gegen andere L- oder D-Aminosäuren ausgetauscht sein können, wobei m die oben angegebene Bedeutung hat.

10. Verbindungen nach mindestens einem der vorhergehenden Ansprüche, **dadurch gekennzeichnet, daß** mindestens eine der Aminosäuren (X)ₘ unabhängig voneinander gegen andere, nicht natürliche Aminosäuren oder Aminosäurederivate ausgetauscht sein kann.

11. Verbindungen nach Anspruch 10, **dadurch gekennzeichnet, daß** als nicht natürliche Aminosäuren oder Aminosäurederivate eine der folgenden Verbindungen ausgewählt ist: Naphthalanin, Cyclohexylalanin, Norleucin, Norvalin, α-Aminoadipinsäure, α-Aminobuttersäure, β-Alanin, β-Cyclohexlyalanin, Ornithin, Sarcosin oder δ-Hydroxylysin.

12. Verbindungen nach mindestens einem der vorhergehenden Ansprüche, **dadurch gekennzeichnet, daß** sämtliche Aminosäuren (X)ₘ gegen ihre jeweilige D-Aminosäure ausgetauscht sind.

13. Verbindungen nach Anspruch 8, **dadurch gekennzeichnet, daß** als Fragmente, Teilsequenzen, Derivate oder Analoga des Somatostatins folgende Aminosäuresequenzen ausgewählt sind:

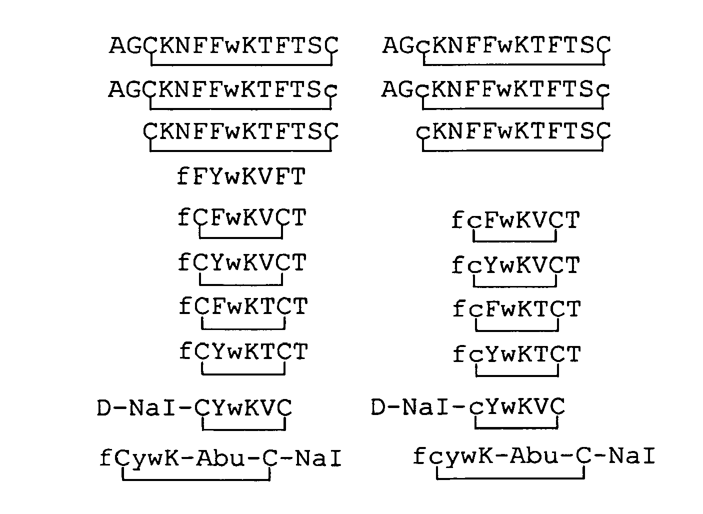

14. Verwendung der Verbindungen nach mindestens einem der vorangehenden Ansprüche zur Herstellung eines Diagnostikums zur In-vivo-Diagnostik von Tumoren, anderen erkrankten Gewebebereichen oder Adenomen mittels optischer Detektionsverfahren, oder zur In-vivo-Fluoreszenzdiagnostik von Tumoren, Tumorzellen und/oder entzündlichen Geweben mittels endoskopischer Verfahren im Gastrointestinaltrakt, Oesophagus, Bronchialtrakt, der Blase oder der Zervix, oder zur In-vivo-Fluoreszenzdiagnostik und/oder Absorptionsdiagnostik von Brusttumoren mittels der optischen Mammographie (Transillumination oder optische Tomographie der Brust).

15. Verwendung der Verbindungen nach Anspruch 1 zur Herstellung eines Diagnostikums zur endoskopischen In-vivo-Fluoreszenzdiagnostik.

16. Optisches Diagnostikum zur In-vivo-Diagnostik erkrankter Gewebebereiche, **dadurch gekennzeichnet, daß** es mindestens eine Verbindung nach Anspruch 1 zusammen mit den üblichen Hilfs- und/oder Trägerstoffen sowie Verdünnungsmitteln enthält.

## Claims

1. Peptide-polymethine-dye conjugates of the formula formula (I):
A¹-(X)ₘ-A² I
in which
X stands for an α-, β- or γ-amino acid with D- or L-configuration, and
m stands for a number from 5 to 30, wherein the resulting amino acid sequence, (X)ₘ, is optionally cyclized in a straight-chain nature or via a disulphide bridge between two cysteines or homocysteines or amidically between the N- and C-terminus, and (X)ₘ is an amino acid sequence of somatostatin or fragments, partial sequences, derivatives or analogues of somatostatin,
A¹ is a hydrogen atom, an acetyl radical or an alkyl radical with up to 10 C-atoms, which is optionally substituted with 1 to 3 carboxy groups and/or 1 to 6 hydroxy groups, or a poly(oxyethylene) radical with 2 to 30 -CH₂CH₂O units, or a dye molecule selected from polymethine dyes which have at least one absorption maximum in the range of 380 to 1200 nm,
A² is a hydroxy group, an amino group or a dye molecule selected from polymethine dyes which have at least one absorption maximum in the range of 380 to 1200 nm,
under the condition that at least one of radicals A¹ or A² is a dye molecule selected from polymethine dyes which have at least one absorption maximum in the range of 380 to 1200 nm,
wherein for the case that A¹ and/or A² is a dye molecule selected from polymethine dyes which have at least one absorption maximum in the range of 380 to 1200 nm, A¹ is linked to the N-terminal amino group and A² is linked to an amino group of the amino acid lysine or to a hydroxy group of the amino acid serine in any position within the amino acid sequence (X)ₘ,
wherein dye molecule selected from polymethine dyes represents a cyanine dye of the general formulae II 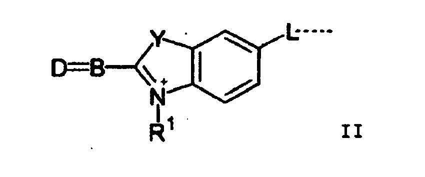 in which
D is a fragment of one of the general the general formulae III to VI, wherein the position that is identified by a star indicates the linkage with B 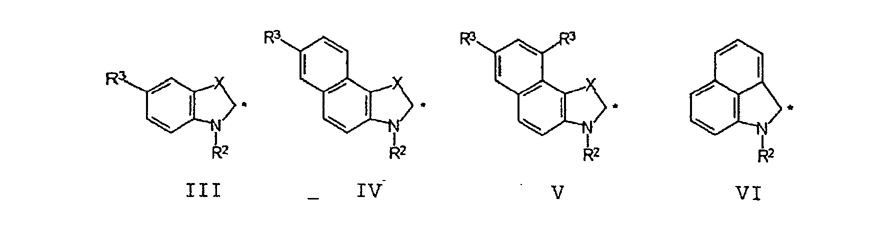 B is a fragment of one of formulae VII to XII 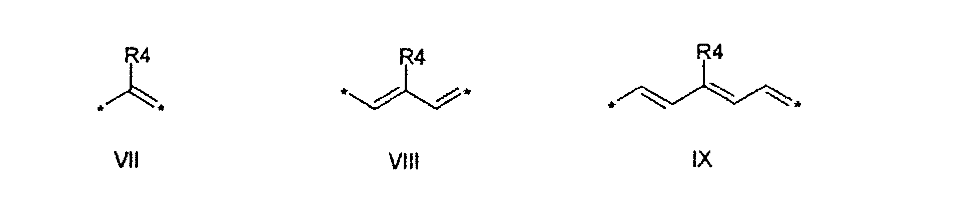 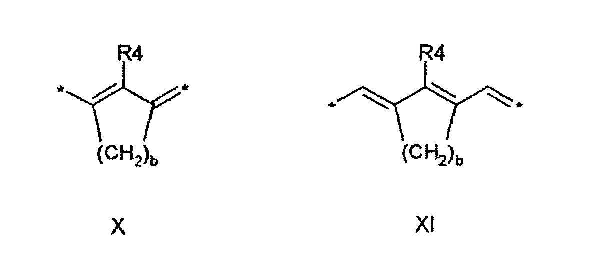 R¹ and R² are E¹, R³ is selected from the group consisting of fluorine, chlorine, bromine and iodine atoms, the nitro group and the radicals -COOE¹, -CONE¹E², -NHCOE¹, -NHCONHE¹, -NE¹E², -OE¹, -OSO₃E¹, -SO₃E¹, -SO₂NHE¹, and -E¹,
where E¹ and E², independently of one another, are a hydrogen atom, a C₁-C₄ sulphoalkyl chain, a saturated or unsaturated, branched or straight-chain C₁-C₅₀ alkyl chain, where the chain or parts of the chain optionally can form one or more aromatic or saturated cyclic C₅-C₆ units or bicyclic C₁₀ units, and wherein the C₁-C₅₀ alkyl chain is interrupted by 0 to 15 oxygen atoms and/or by 0 to 3 carbonyl groups and/or is substituted with 0 to 5 hydroxy groups,
R⁴ is a hydrogen atom, a fluorine, chlorine, bromine, iodine atom or a branched or straight-chain C₁-C₁₀ alkyl chain,
b is the number 2 or 3,
X and Y, independently of one another, are O, S, Se, -CH=CH- or C(CH₃)₂,
L is a group of one of the formulae below 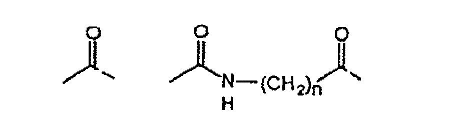 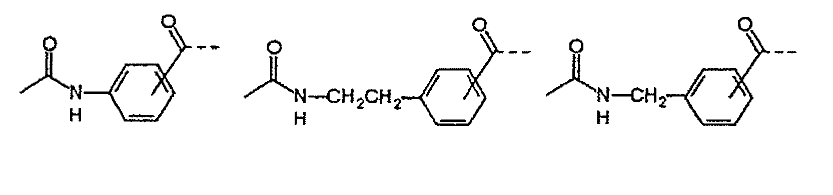
in which
n is a number from 1 to 10
and the physiologically compatible salts thereof.

2. Compounds according to Claim 1, **characterized in that** the dye molecule A¹ and/or A² is an indocarbocyanine dye, an indodicarbocyanine dye or an indotricarbocyanine dye.

3. Compounds according to Claim 2, **characterized in that** the dye molecule A¹ and/or A² is an indocarbocyanine dye, an indodicarbocyanine dye or an indotricarbocyanine dye of one of the general formulae XIII or XIV 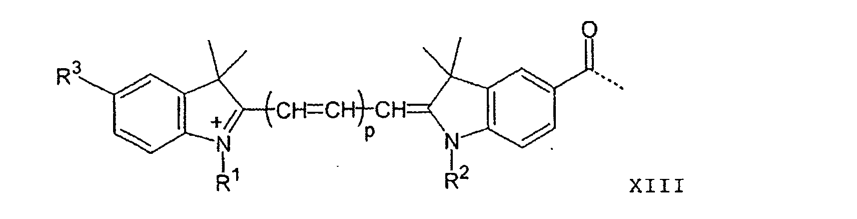 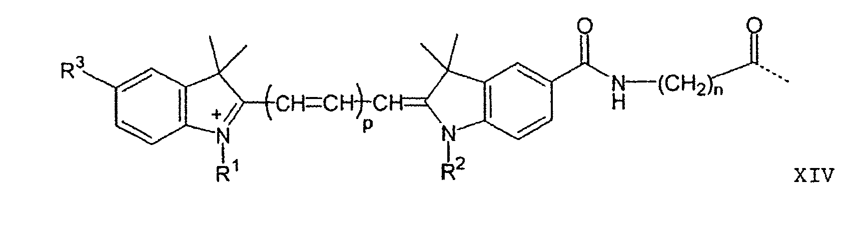 in which
p is 1, 2 or 3,
n is 1, 2, 3, 4 or 10,
R¹ and R², independently of one another, are a 4-sulphobutyl, 3-sulphopropyl, 2-sulphoethyl, 3-methyl-3-sulphopropyl, methyl, ethyl or propyl radical, and R³ is hydrogen, a chlorine, bromine, iodine atom or a nitro group or a radical -COOE¹, -CONE¹E², -NHCOE¹, -NHCONHE¹, -NE¹E², -OE¹, -OSO₃E¹, -SO₃E¹, -SO₂NHE¹,
wherein E¹ and E², independently of one another, are a hydrogen atom or a methyl or ethyl radical or a C₃-C₆ alkyl radical, which is interrupted by 0 to 2 oxygen atoms and/or by 0 to 1 carbonyl groups and/or is substituted with 0 to 5 hydroxy groups, or E¹ and E² are a poly(oxyethylene)glycol radical with 2 to 30 -CH₂CH₂O units.

4. Compounds according to Claim 2, **characterized in that** the dye molecule A¹ and/or A² is an indocarbocyanine, indodicarbocyanine or indotricarbocyanine dye of one of the general formulae XIII or XIV 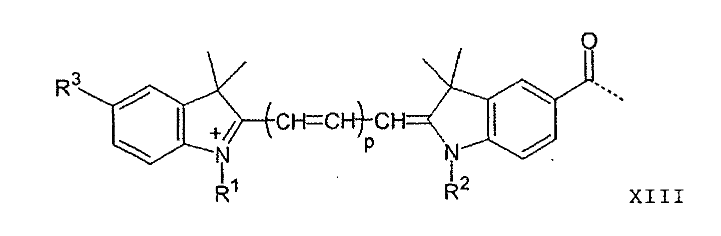 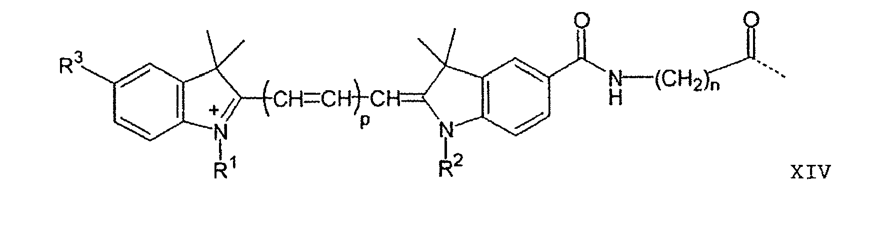 in which
p is 1, 2 or 3,
n is 1, 2 or 4,
R¹ and R², independently of one another, are a 4-sulphobutyl or 3-sulphopropyl radical,
R³ is hydrogen or a radical -COOE¹ or -CONHE¹,
wherein E¹ is a hydrogen atom or a methyl or ethyl radical or a C₃-C₆ alkyl radical, which is interrupted by 0 to 2 oxygen atoms and/or by 0 to 1 carbonyl group and/or is substituted with 0 to 5 hydroxy groups.

5. Compounds according to Claim 2 **characterized in that** the dye molecule A¹ and/or A² is an indotricarbocyanine dye of one of the general formulae XV or XVI: 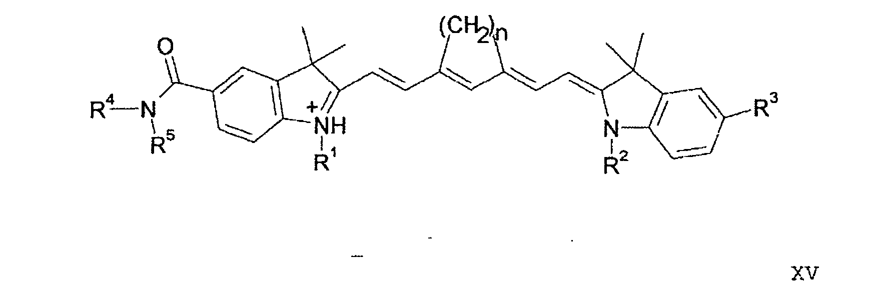 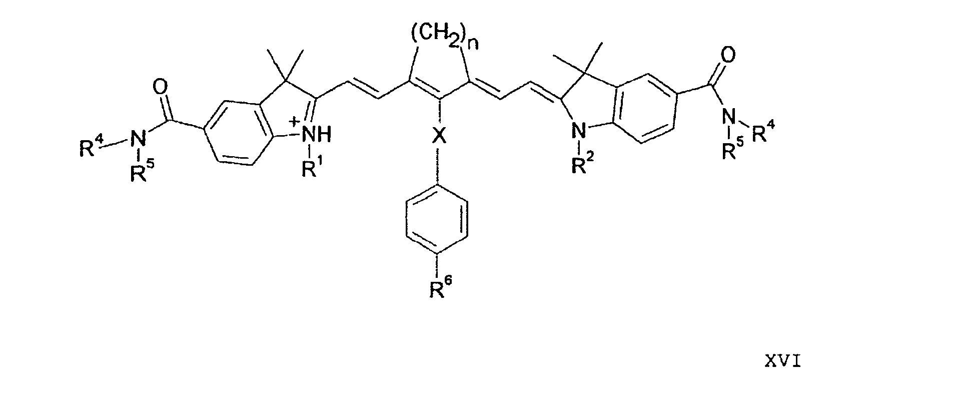 in which
n is 2 or 3,
R¹ and R², independently of one another, are a 4-sulphobutyl, 3-sulphopropyl or 2-sulphoethyl radical,
R³ is a radical -CONH-peptide, -CONH-(CH₂)ₘ-CONH-peptide, -CONH-(CH₂)ₙ-NH-CS-NH-peptide or -CONH-(CH₂)ₙ-NHCO-CH₂-peptide with m = 1 to 10 and n = 2 or 3, or R³ is a group of the following formula: 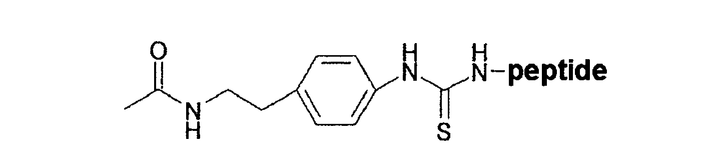 R⁴ and R⁵, independently of one another, are a hydrogen atom, a methyl radical or a hydroxylated alkyl radical,
R⁶ is one of the following groups:
-(CH₂)ₘ-CONH-peptide with m = 0 to 2, or
-(CH₂)ₘ-NH-CS-NH-peptide with m = 0 to 2,
and X stands for an oxygen atom or a sulphur atom.

6. Compounds according to Claim 2, **characterized in that** the dye molecule A¹ and/or A² is an indotricarbocyanine dye of the general formula XVII 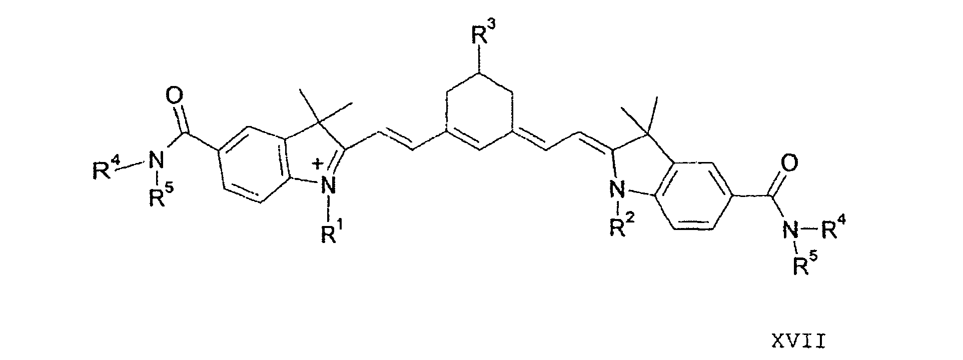 in which
R¹ and R², independently of one another, are a 4-sulphobutyl radical, 3-sulphopropyl radical, or 2-sulphoethyl radical,
R³ is a radical -CONH-peptide, -NH-CS-NH-peptide or -CONH-(CH₂)ₙ-NHCO-CH₂-peptide with n = 2 or 3, or R³ is a group of the following formula: 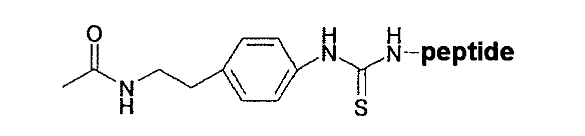 and R⁴ and R⁵, independently of one another, are a hydrogen atom, a methyl radical or a hydroxylated alkyl radical.

7. Compounds according to Claim 5 or 6, **characterized in that** the hydroxylated alkyl radicals are 2-hydroxyethyl, 3-hydroxypropyl, 2,3-dihydroxypropyl, 1,3-dihydroxy-2-propyl, 2,3,4-trihydroxybutyl, 1,3,4-trihydroxy-2-butyl, or 2,3,4,5,6-pentahydroxyhexyl.

8. Compounds according to Claim 1, **characterized in that** (X)ₘ is the amino acid sequence of somatostatin corresponding to 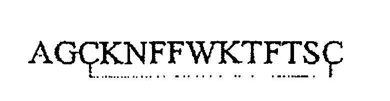 or fragments, partial sequences, derivatives or analogues of somatostatin, consisting of 5 to 20 amino acids.

9. Compounds according to at least one of the preceding claims, **characterized in that** 2 to m amino acids, independently of one another, are optionally exchanged for their respective D-amino acid or for other L- or D-amino acids, wherein m has the above-indicated meaning.

10. Compounds according to at least one of the preceding claims, **characterized in that** at least one of the amino acids (X)ₘ, independently of one another, is optionally exchanged for other, non-natural amino acids or amino acid derivatives.

11. Compounds according to Claim 10, **characterized in that,** as non-natural amino acids or amino acid derivatives, one of the following compounds is selected: naphthalanine, cyclohexylalanine, norleucine, norvaline, α-aminoadipic acid, α-aminobutyric acid, β-alanine, β-cyclohexylalanine, ornithine, sarcosine and δ-hydroxylysine.

12. Compounds according to at least one of the preceding claims, **characterized in that** all amino acids (X)ₘ are exchanged for their respective D-amino acids.

13. Compounds according to Claim 8, **characterized in that**, as fragments, partial sequences, derivatives or analogues of somatostatin, the following amino acid sequences are selected:

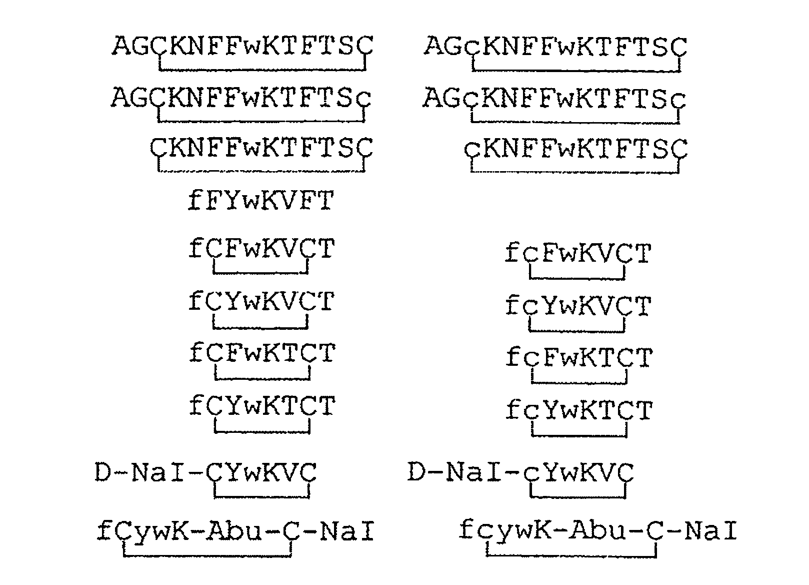

14. Use of the compounds according to at least one of the preceding claims for preparing a diagnostic agent for in-vivo diagnosis of tumours, other diseased tissue areas or adenomas using optical detection processes, or for in-vivo fluorescence diagnosis of tumours, tumour cells and/or inflammatory tissues using endoscopic processes in the gastrointestinal tract, oesophagus, bronchial tract, bladder or cervix, or for in-vivo fluorescence diagnosis and/or absorption diagnosis of breast tumours using optical mammography (transillumination or optical tomography of the breast).

15. Use of the compounds according to Claim 1 in the manufacture of a diagnostic agent for endoscopic in-vivo fluorescence diagnostics.

16. Optical diagnostic agent for in-vivo diagnosis of diseased tissue areas, **characterized in that** it comprises at least one compound according to Claim 1 together with common adjuvants and/or vehicles as well as diluents.

## Revendications

1. Conjugués peptide-colorant de type polyméthine de formule générale (I)
A¹-(X)ₘ-A² I
où
X représente un acide α, β ou γ-aminé présentant la configuration D ou L et
m vaut un nombre de 5 à 30,
la séquence d'acides aminés obtenue (X)ₘ présentant une nature linéaire ou pouvant être cyclisée via un pont disulfure entre deux cystéines ou homocystéines ou amidiquement entre une terminaison N et C et représentant la séquence d'acides aminés de la somatostatine ou des fragments, des séquences partielles ou des analogues de la somatostatine,
A¹ représente un atome d'hydrogène, un radical acétyle ou un radical alkyle comprenant jusqu'à 10 atomes de carbone, qui peut le cas échéant être substitué par 1 à 3 groupes carboxy et/ou 1 à 6 groupes hydroxy ou un radical poly(oxyéthylène) comprenant 2 à 30 unités -CH₂CH₂O- ou une molécule de colorant de la classe des colorants de type polyméthine qui présente au moins un maximum d'absorption dans la plage de 380 à 1200 nm,
A² représente un groupe hydroxy, un groupe amino ou une molécule de colorant de la classe des colorants de type polyméthine qui présente au moins un maximum d'absorption dans la plage de 380 à 1200 nm,
à condition qu'au moins un des radicaux A¹ ou A² représente une molécule de colorant de la classe des colorants de type polyméthine qui présente au moins un maximum d'absorption dans la plage de 380 à 1200 nm,
où, pour le cas où A¹ et/ou A² représenteraient une molécule de colorant de la classe des colorants de type polyméthine, qui présente au moins un maximum d'absorption dans la plage de 380 à 1200 nm, A¹ est lié au groupe amino N-terminal et A² est lié à un groupe amino de l'acide aminé lysine ou à un groupe hydroxy de l'acide aminé sérine en une position quelconque dans la séquence d'acides aminés (X)ₘ,
où la molécule de colorant de la classe des colorants de type polyméthine représente un colorant de type cyanine de formule générale II, 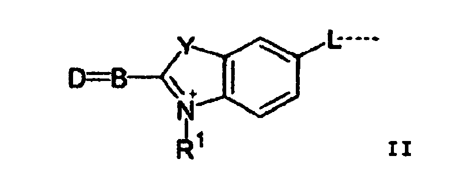 où
D représente un fragment selon les formules générales III à VI, la position **caractérisée par** un astérisque signifiant la liaison avec B, 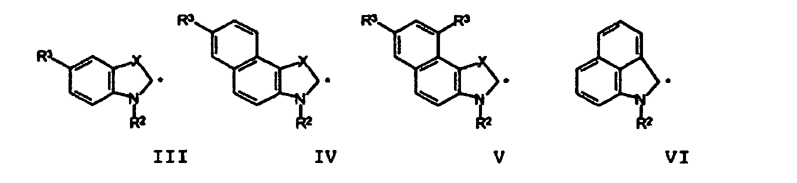
B représente un fragment selon les formules générales VII à XII 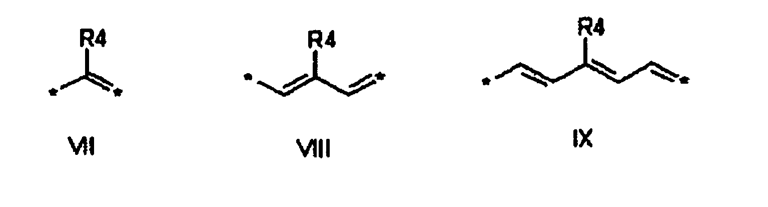 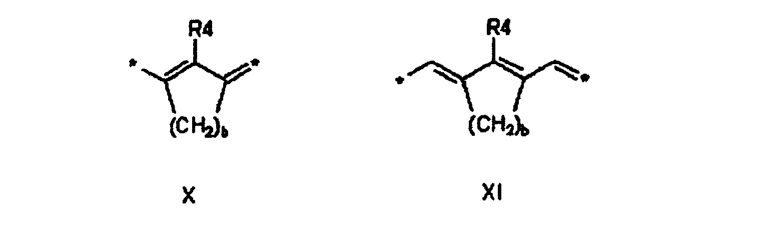
R¹ et R² représentent E¹, R³ représente un atome de fluor, de chlore, de brome, d'iode ou un groupe nitro ou un radical -COOE¹, -CONE¹E², -NHCOE¹, -NHCONHE¹, -NE¹E², -OE¹, -OSO₃E¹, -SO₃E¹, -SO₂NHE¹, -E¹,
où E¹ et E² représentent, indépendamment l'un de l'autre, un atome d'hydrogène, une chaîne C₁-C₄-sulfoalkyle, une chaîne C₁-C₅₀-alkyle saturée ou insaturée, ramifiée ou linéaire, la chaîne ou des parties de cette chaîne pouvant le cas échéant former une ou plusieurs unités aromatiques ou saturées, cycliques en C₅-C₆ ou bicycliques en C₁₀, et où la chaîne C₁-C₅₀-alkyle est interrompue par 0 à 15 atomes d'oxygène et/ou par 0 à 3 groupes carbonyle et/ou substituée par 0 à 5 groupes hydroxy,
R⁴ représente un atome d'hydrogène, un atome de fluor, de chlore, de brome, d'iode ou une chaîne C₁-C₁₀-alkyle ramifiée ou linéaire,
b vaut un nombre égal à 2 ou 3,
X et Y signifient, indépendamment l'un de l'autre, O, S, Se, -CH=CH- ou C(CH₃)₂,
L représente un groupe selon les formules suivantes 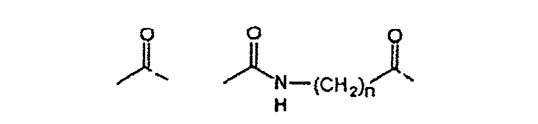 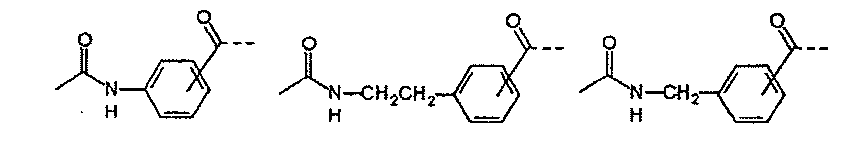 où
n signifie un nombre de 1 à 10,
et leurs sels physiologiquement acceptables.

2. Composés selon la revendication 1, **caractérisés en ce que** la molécule de colorant A¹ et/ou A² représente un colorant de type indocarbocyanine, indodicarbocyanine ou indotricarbocyanine.

3. Composés selon la revendication 2, **caractérisés en ce que** la molécule de colorant A¹ et/ou A² représente un colorant de type indocarbocyanine, indodicarbocyanine ou indotricarbocyanine de formule générale XIII ou XIV 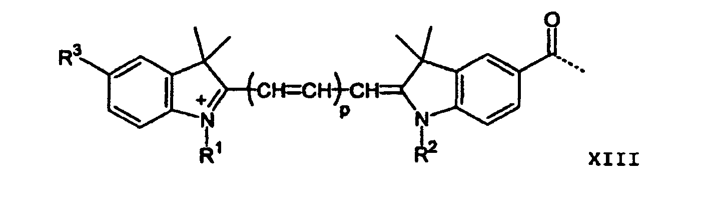 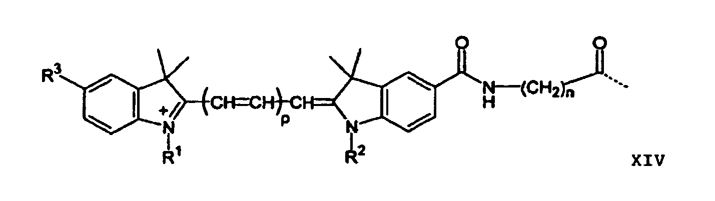 où
p vaut 1, 2 ou 3,
n vaut 1, 2, 3, 4 ou 10,
R¹ et R² représentent, indépendamment l'un de l'autre, un radical 4-sulfobutyle, 3-sulfopropyle, 2-sulfoéthyle, 3-méthyl-3-sulfopropyle, méthyle, éthyle ou propyle, et
R¹ représente hydrogène, un atome de chlore, de brome, d'iode ou un groupe nitro ou un radical -COOE¹, -CONE¹E², -NHCOE¹, -NHCONHE¹, -NE¹E², -OE¹, -OSO₃E¹, -SO₃E¹, -SO₂NHE¹,
où E¹ et E² représentent, indépendamment l'un de l'autre, un atome d'hydrogène ou un radical méthyle, éthyle ou C₃-C₆-alkyle, qui est interrompu par 0 à 2 atomes d'oxygène et/ou par 0 à 1 groupe carbonyle et/ou substitué par 0 à 5 groupes hydroxy ou un radical poly(oxyéthylène)glycol comprenant 2 à 30 unités -CH₂CH₂O-.

4. Composés selon la revendication 2, **caractérisés en ce que** la molécule de colorant A¹ et/ou A² représente un colorant de type indocarbocyanine, indodicarbocyanine ou indotricarbocyanine de formule générale XIII ou XIV 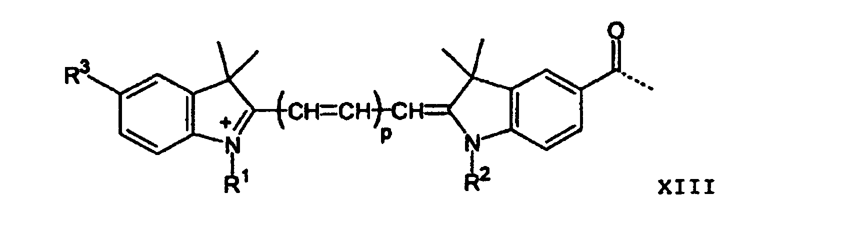 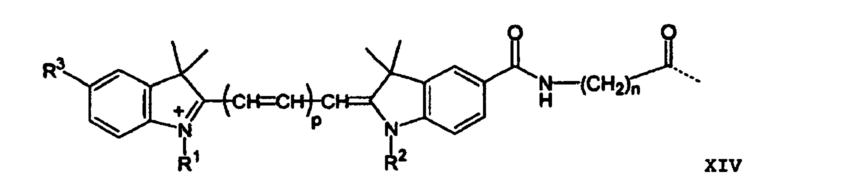 où
p vaut 1, 2 ou 3,
n vaut 1, 2 ou 4,
R¹ et R² représentent, indépendamment l'un de l'autre, un radical 4-sulfobutyle ou 3-sulfopropyle,
R³ représente hydrogène ou un radical -COOE¹ ou -CONHE¹,
où E¹ signifie un atome d'hydrogène ou un radical méthyle, éthyle ou C₃-C₆-alkyle, qui est interrompu par 0 à 2 atomes d'oxygène et/ou par 0 à 1 groupe carbonyle et/ou substitué par 0 à 5 groupes hydroxy.

5. Composés selon la revendication 2, **caractérisés en ce que** la molécule de colorant A¹ et/ou A² représente un colorant de type indotricarbocyanine de formule générale XV ou XVI 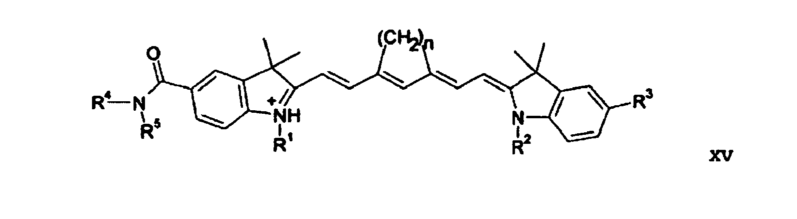 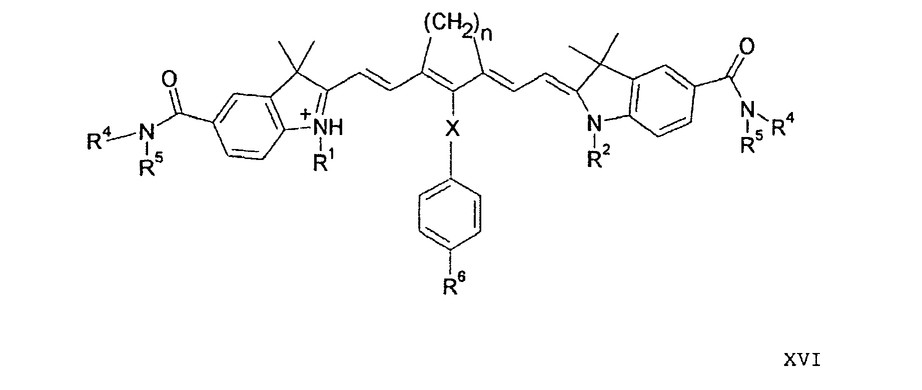 où
n vaut 2 ou 3,
R¹ et R² représentent, indépendamment l'un de l'autre, un radical 4-sulfobutyle, 3-sulfopropyle ou 2-sulfoéthyle,
R³ représente un radical -CONH-peptide, -CONH-(CH₂)ₘ-CONH-peptide, -CONH-(CH₂)ₙ-NH-CS-NH-peptide ou -CONH-(CH₂)ₙ-NHCO-CH₂-peptide avec m = 1 à 10 et n = 2 ou 3,
ou un groupe ci-dessous : 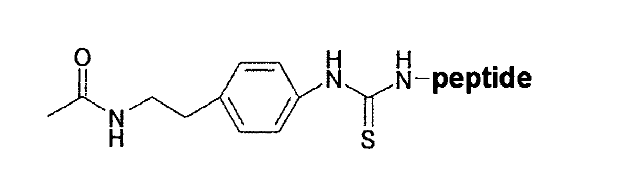
R⁴ et R⁵ représentent, indépendamment l'un de l'autre, un atome d'hydrogène, un radical méthyle ou un radical alkyle hydroxylé,
R⁶ représente un des groupes suivants :
-(CH₂)ₘ-CONH-peptide avec m = 0 à 2,
-(CH₂)ₘ-NH-CS-NH-peptide avec m = 0 à 2,
et X représente un atome d'oxygène ou un atome de soufre.

6. Composés selon la revendication 2, **caractérisés en ce que** la molécule de colorant A¹ et/ou A² représente un colorant de type indotricarbocyanine de formule générale XVII 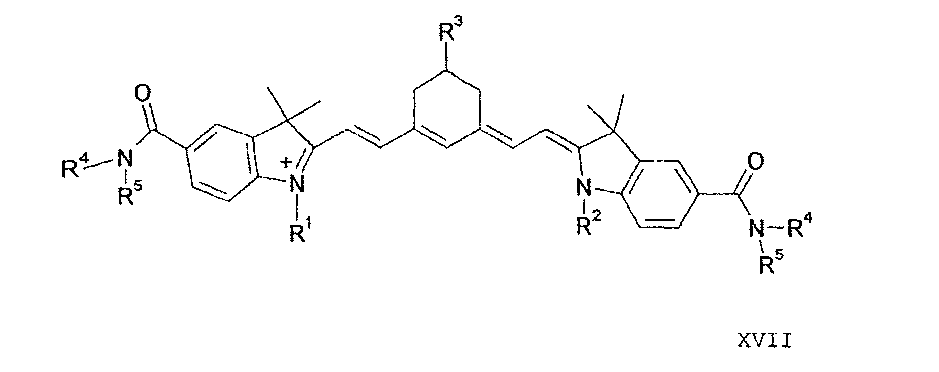 où
R¹ et R² représentent, indépendamment l'un de l'autre, un radical 4-sulfobutyle, 3-sulfopropyle ou 2-sulfoéthyle,
R³ représente un radical -CONH-peptide, -NH-CS-NH-peptide ou -CONH-(CH₂)ₙ-NHCO-CH₂-peptide avec n = 2 ou 3 ou un groupe suivant : 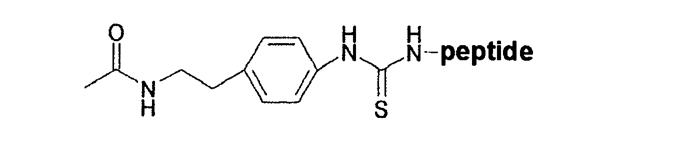
et R⁴ et R⁵ représentent, indépendamment l'un de l'autre, un atome d'hydrogène, un radical méthyle ou un radical alkyle hydroxylé.

7. Composés selon la revendication 5 ou 6, **caractérisés en ce que** les radicaux alkyle hydroxylés représentent 2-hydroxyéthyle, 3-hydroxypropyle, 2,3-dihydroxypropyle, 1,3-dihydroxy-2-propyle, 2,3,4-trihydroxybutyle, 1,3,4-trihydroxy-2-butyle, 2,3,4,5,6-pentahydroxyhexyle.

8. Composés selon la revendication 1, **caractérisés en ce que** (X)ₘ représente la séquence d'acides aminés de la somatostatine selon 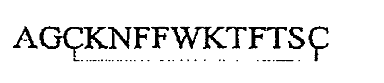 ou des fragments, des séquences partielles, des dérivés ou des analogues de la somatostatine, constitués par 5 à 20 acides aminés.

9. Composés selon au moins l'une quelconque des revendications précédentes, **caractérisés en ce que** 2 à m acides aminés, indépendamment l'un de l'autre, peuvent être remplacés par à chaque fois leur acide aminé D ou par d'autres acides aminés L ou D, m ayant la signification indiquée ci-dessus.

10. Composés selon au moins l'une quelconque des revendications précédentes, **caractérisés en ce qu'**au moins un des acides aminés (X)ₘ, indépendamment l'un de l'autre, peut être remplacé par d'autres acides aminés non naturels ou des dérivés d'acides aminés non naturels.

11. Composés selon la revendication 10, **caractérisés en ce qu'**on choisit comme acides aminés non naturels ou dérivés d'acides aminés non naturels un des composés suivants : naphtalanine, cyclohexylalanine, norleucine, norvaline, acide α-aminoadipique, acide α-aminobutyrique, β-alanine, β-cyclohexylalanine, ornithine, sarcosine ou δ-hydroxylysine.

12. Composés selon au moins l'une quelconque des revendications précédentes, **caractérisés en ce que** tous les acides aminés (X)ₘ sont remplacés par à chaque fois leur acide aminé D.

13. Composés selon la revendication 8, **caractérisés en ce qu'**on choisit comme fragments, séquences partielles, dérivés ou analogues de la somatostatine les séquences d'acides aminés suivantes :

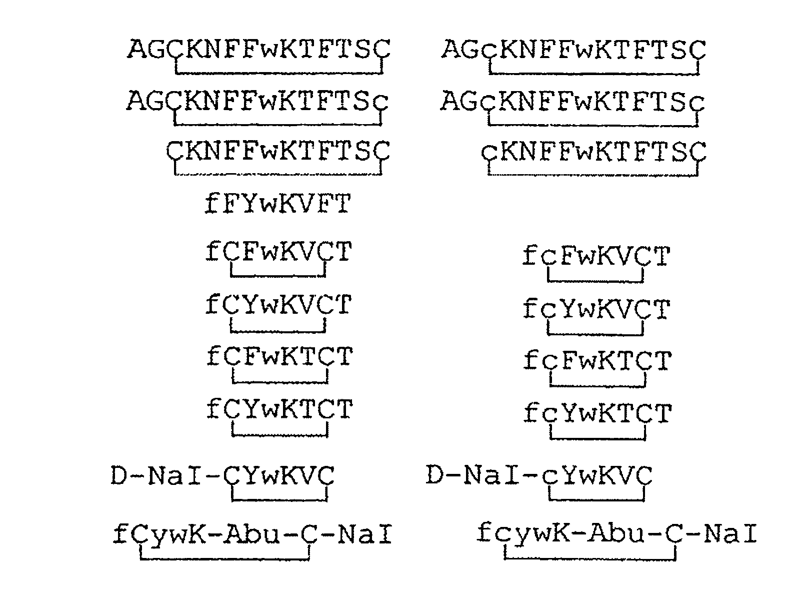

14. Utilisation des composés selon au moins l'une quelconque des revendications précédentes pour la préparation d'un agent diagnostique pour le diagnostic in vivo de tumeurs, d'autres zones tissulaires malades ou d'adénomes au moyen d'un procédé de détection optique, ou pour le diagnostic in vivo par fluorescence de tumeurs, de cellules tumorales et/ou de tissus enflammés par des procédés endoscopiques dans le tractus gastro-intestinal, l'oesophage, le tractus bronchique, la vessie ou le col de l'utérus, ou pour le diagnostic in vivo par fluorescence et/ou le diagnostic par absorption de tumeurs du sein au moyen de la mammographie optique (transillumination ou tomographie optique du sein).

15. Utilisation des composés selon la revendication 1 pour la préparation d'un diagnostic destiné au diagnostic in vivo par fluorescence endoscopique.

16. Agent diagnostique optique pour le diagnostic in vivo de zones tissulaires malades, **caractérisé en ce qu'**il contient au moins un composé selon la revendication 1 avec les adjuvants et/ou substances support ainsi que les diluants usuels.
